# EUROPEAN PATENT APPLICATION

(11) **EP 4 696 233 A1**
(43) Date of publication of application: **18.02.2026**
(21) Application number: 24789037.9
(22) Date of filing: 11.04.2024
(51) Int. Cl.: A61B 5/145, A61B 5/00, A61B 5/155, A61B 5/157

(54) **SENSING DEVICE AND APPLICATOR ASSEMBLY**

(30) Priority: 14.04.2023 KR 20230049668; 09.04.2024 KR 20240047842
(71) Applicant: i-Sens, Inc., Seoul 06646 (KR)
(72) Inventor: CHOI, Wonseok, Seoul 06646 (KR); CHOI, Hyunho, Seoul 06646 (KR); PARK, Kyeongseok, Seoul 06646 (KR)
(74) Representative: HGF
(86) International application number: PCT/KR2024/004825
(87) International publication number: WO 2024/215095

(57) **Abstract**

A sensing device according to one aspect of the present disclosure is a sensing device configured to be inserted and attached to a user through an applicator including a sensor unit including a transcutaneous sensor, a sensor unit housing configured to accommodate the sensor inside while exposing one end of the sensor through a lower surface, and a base leg protruding from the lower surface of the sensor unit housing, and a transmitter disposed at a lower part of the sensor unit within the applicator, wherein the transmitter includes a transmitter housing having a mounting portion formed on an upper surface and configured to accommodate the sensor unit, and a coupling protrusion disposed on the mounting portion and configured to be engaged with the base leg.

## Description

### Technical Field

The present disclosure relates to a sensing device and an applicator assembly.

### Background Art

Recent advancements in medical technology have led to the development and sale of various medical devices that may be used directly by non-medical users. Examples of such medical devices include blood glucose meters, which users use to directly monitor their own biometric information, such as blood glucose levels.

Blood glucose meters include lancet-type blood glucose meters and continuous glucose monitoring systems (CGMS).

Because the lancet-type blood glucose meter requires the user to draw blood, it may be difficult to monitor immediately for hypoglycemia.

Although CGMS may increase the user's inconvenience when used, its use is increasing because it may monitor the user's biometric information relatively immediately by inserting and attaching a sensing device to the user's skin.

Prior art documents of the present disclosure include Korean Patent Application Publication No. 2020-0127097.

### Disclosure of the Invention

### Technical Goals

One object of embodiments of the present disclosure is to provide a sensing device and applicator assembly that allows a user to easily attach the sensing device to the skin.

Another object of embodiments of the present disclosure is to provide a sensing device and applicator assembly that allows for the easy coupling of a sensor unit and a transmitter, which are disposed to be spaced apart from each other within the applicator.

### Technical Solutions

According to an aspect of the present disclosure, there is provided a sensing device configured to be inserted and attached to a user through an applicator including a sensor unit including a transcutaneous sensor, a sensor unit housing configured to accommodate the sensor inside while exposing one end of the sensor through a lower surface, and a base leg protruding from the lower surface of the sensor unit housing, and a transmitter disposed at a lower part of the sensor unit within the applicator, wherein the transmitter includes a transmitter housing having a mounting portion formed on an upper surface and configured to accommodate the sensor unit, and a coupling protrusion disposed on the mounting portion and configured to be engaged with the base leg.

Here, an exposure hole configured to expose an inside of the transmitter housing may be formed on a bottom surface of the mounting portion, and the coupling protrusion may include a support portion in contact with the bottom surface of the mounting portion, and a protrusion portion extending from the support portion to protrude from an inner wall of the exposure hole.

In addition, a distance by which a lower region of the protrusion portion protrudes from the inner wall of the exposure hole may be longer than a distance by which an upper region of the protrusion portion protrudes from the inner wall of the exposure hole.

In addition, the base leg may include leg portions respectively in contact with the lower surface of the sensor unit housing and spaced apart from each other, and a connecting portion configured to connect lower end portions of the leg portions spaced apart from each other and define a fastening hole together with the leg portions.

In addition, a thickness of an upper end portion of the connecting portion connected to the leg portions may be thicker than a thickness of a lower end portion of the connecting portion.

In addition, a thickness of the connecting portion may increase from the lower end portion of the connecting portion toward the upper end portion of the connecting portion.

According to another aspect of the present disclosure, there is provided a sensing device including a sensor unit including a transcutaneous sensor, a sensor unit housing configured to accommodate the sensor inside while exposing one end of the sensor through a lower surface, and a base leg protruding from the lower surface of the sensor unit housing, and a transmitter coupled with the sensor unit, wherein the transmitter includes a transmitter housing, a mounting portion formed on the transmitter housing and configured to accommodate the sensor unit housing, an insertion hole penetrating the transmitter housing and configured to accommodate at least a portion of one end of the sensor, and a coupling protrusion disposed within the mounting portion and configured be engaged with the base leg.

Here, the base leg may include leg portions respectively in contact with the lower surface of the sensor unit housing and spaced apart from each other, and a connecting portion configured to connect lower end portions of the leg portions spaced apart from each other and define a fastening hole together with the leg portions, and at least a portion of the coupling protrusion may be inserted into the fastening hole.

In addition, an exposure hole configured to accommodate the base leg may be formed on a bottom surface of the mounting portion, and a distance from one surface of the leg portion facing an inner wall of the exposure hole to the inner wall of the exposure hole may be shorter than a distance from one surface of the connecting portion facing the inner wall of the exposure hole to the inner wall of the exposure hole.

In addition, an exposure hole configured to accommodate the base leg may be formed on a bottom surface of the mounting portion, and the coupling protrusion may include a support portion in contact with the bottom surface of the mounting portion, and a protrusion portion extending from the support portion to protrude from an inner wall of the exposure hole.

In addition, a distance by which the protrusion portion protrudes from an inner wall of the exposure hole may be longer at a lower side of the inner wall of the exposure hole than at an upper side of the inner wall of the exposure hole.

In addition, the sensing device may further include an adhesive layer adhered to each of a bottom surface of the mounting portion and a lower surface of the sensor unit housing.

### Advantageous Effects

According to embodiments of the present disclosure, a user may easily attach a sensing device to the skin.

In addition, according to embodiments of the present disclosure, a sensor unit and a transmitter, which are disposed to be spaced apart from each other within an applicator, may be easily coupled.

### Brief Description of Drawings

FIG. 1 is a perspective view schematically illustrating an applicator assembly according to an embodiment of the present disclosure.
FIG. 2 is a diagram schematically illustrating a state in which a sensing device of an applicator assembly is attached to a user's skin according to an embodiment of the present disclosure.
FIG. 3 is a diagram schematically illustrating a state of a sensor unit and a transmitter of a sensing device before being coupled according to an embodiment of the present disclosure.
FIG. 4 is a diagram schematically illustrating a coupled state of a sensing device according to an embodiment of the present disclosure.
FIG. 5 is a diagram schematically illustrating a cross-section along line III-III' of FIG. 4.
FIG. 6 is a diagram schematically illustrating a cross-section along line IV-IV' of FIG. 4.
FIGS. 7 and 8 are diagrams schematically illustrating an exploded view of a sensor unit, respectively.
FIG. 9 is a diagram schematically illustrating a state in which a needle is coupled to a sensor unit.
FIG. 10 is a diagram schematically illustrating a transmitter and a protective sheet.
FIG. 11 is a diagram schematically illustrating an exploded view of an applicator assembly according to an embodiment of the present disclosure.
FIG. 12 is a diagram schematically illustrating a coupled state of FIG. 11, excluding a top cover.
FIG. 13 is a diagram schematically illustrating an exploded view of a base cover, a supporter, and a removing unit.
FIG. 14 is a diagram illustrating a coupled state of a base cover, a supporter, and a removing unit as viewed from above.
FIG. 15 is a diagram schematically illustrating an exploded view of an inner frame, a moving plate, a plunger, a carrier, a needle, a plunger spring, and a needle spring.
FIG. 16 is a diagram schematically illustrating a middle frame as viewed from below.
FIG. 17 is a diagram schematically illustrating a plunger as viewed from below.
FIGS. 18A and 18B are diagrams schematically illustrating an operation of a moving plate and a supporter by pressing a release button.
FIGS. 19A, 19B, and 19C are diagrams schematically illustrating an operation process of a supporter by a movement of a moving plate as viewed from above, respectively.
FIG. 20 is a diagram schematically illustrating a removing unit and a protective sheet applied to the present embodiment.
FIGS. 21 to 26 are diagrams schematically illustrating a manipulation method of an applicator assembly according to an embodiment of the present disclosure, wherein FIGS. 21, 22, and 24 are each diagrams schematically illustrating a cross-section along a line corresponding to line I-I' of FIG. 1, and FIGS. 23, 25, and 26 are each drawings schematically illustrating a cross-section along a line corresponding to line II-II' of FIG. 1.

### Best Mode for Carrying Out the Invention

The terms used in the present application are used merely to describe particular embodiments and are not intended to limit the present disclosure. Singular expressions include plural expressions unless the context clearly indicates otherwise. In the present application, it should be understood that terms such as "comprise", "include", or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof. In addition, throughout the specification, "on" means located above or below the target part, and does not necessarily mean located on the upper side based on the direction of gravity.

In addition, the term "coupling" is used as a concept that includes not only the case where each component is physically in direct contact with each other, but also the case where another component is interposed between each component, and each component is in contact with the other component.

In addition, the term "detachment" is used as a concept that means that two or more components that are coupled in a state where relative movement does not occur between them move relative to each other by the manipulation of a user or the like within the normal range of use.

Since the size and thickness of each component shown in the drawings are arbitrarily shown for the convenience of description, the present disclosure is not necessarily limited to what is shown.

Hereinafter, a sensing device and an applicator assembly according to an embodiment of the present disclosure will be described in detail with reference to the accompanying drawings, and in describing with reference to the accompanying drawings, identical or corresponding components will be assigned the same drawing numbers and overlapping descriptions thereof will be omitted.

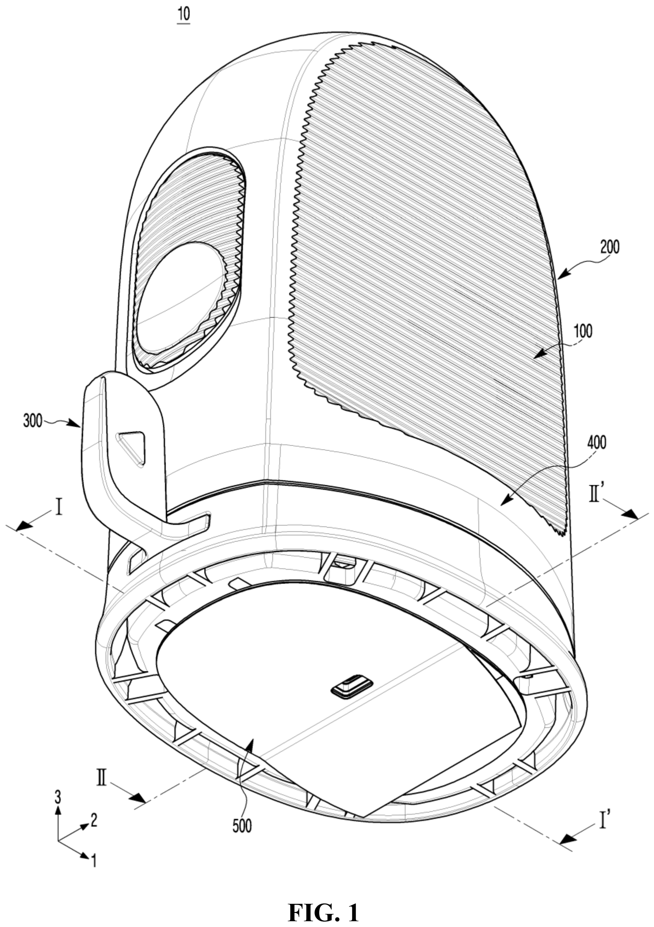

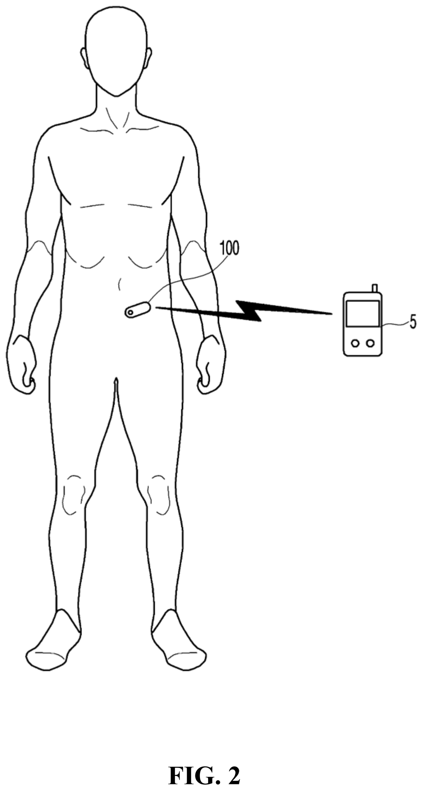

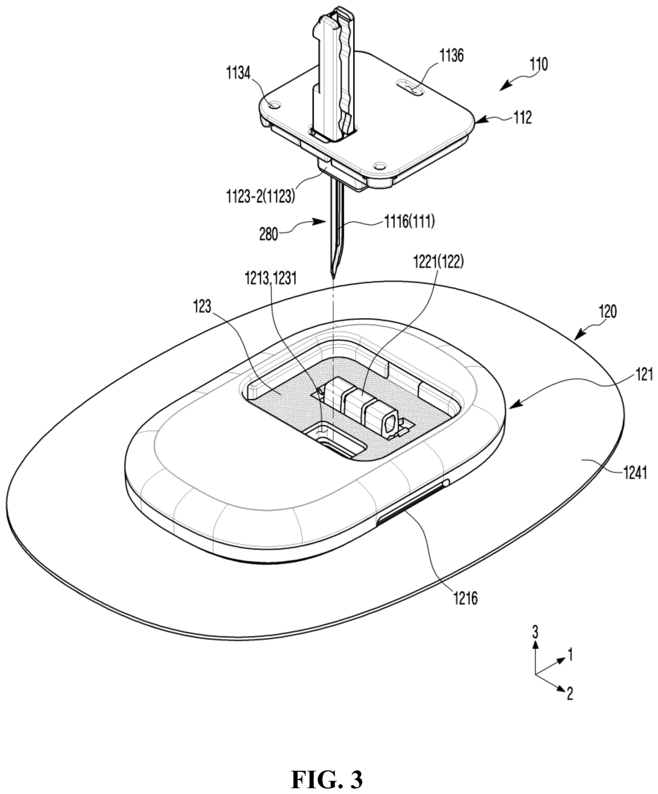

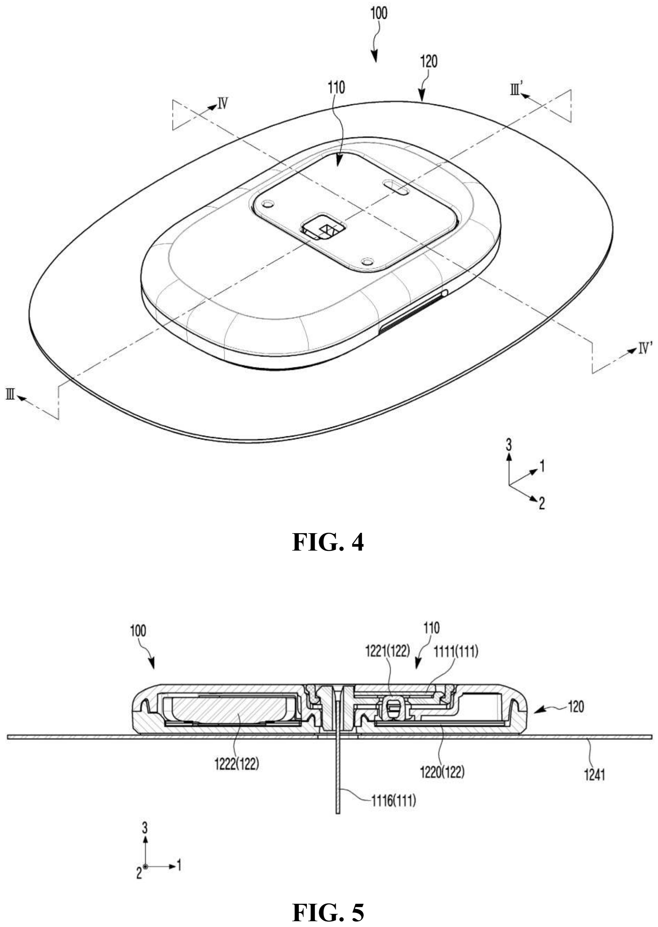

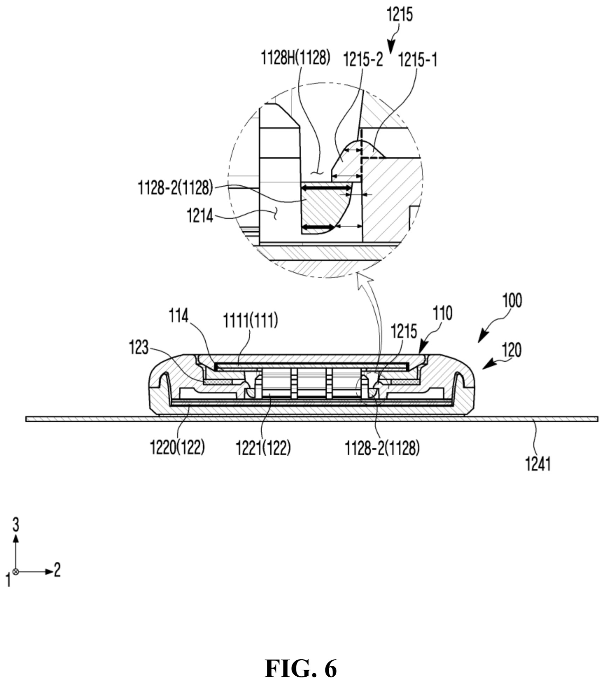

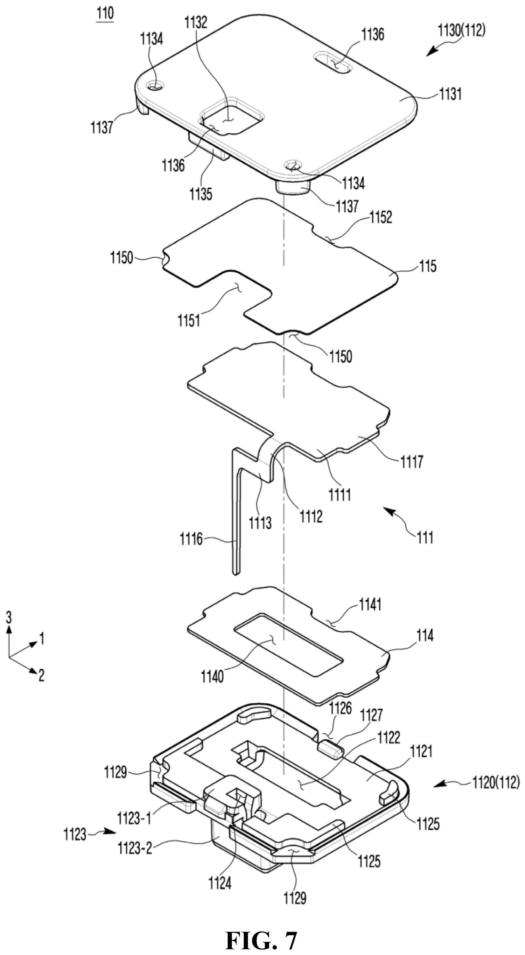

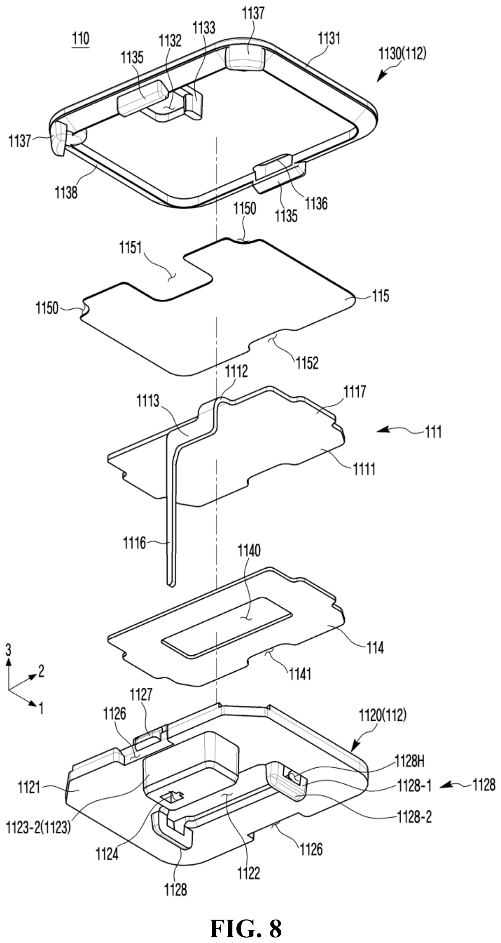

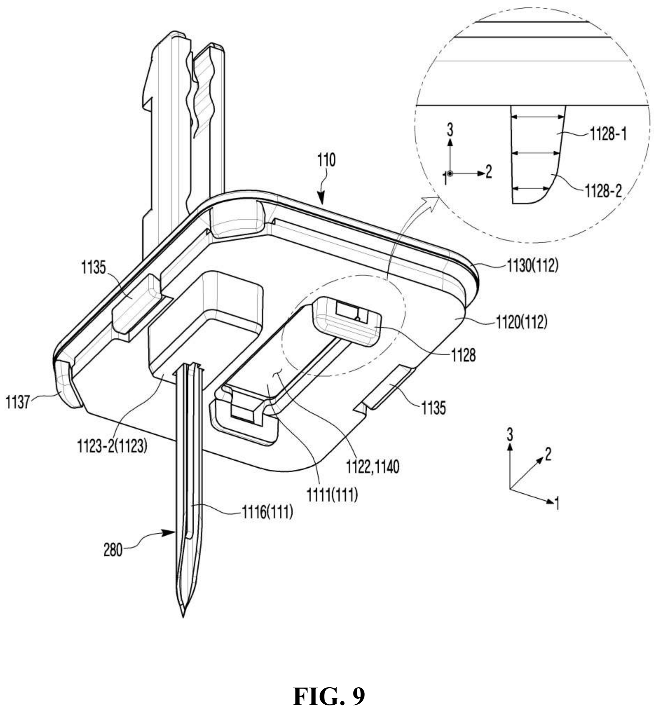

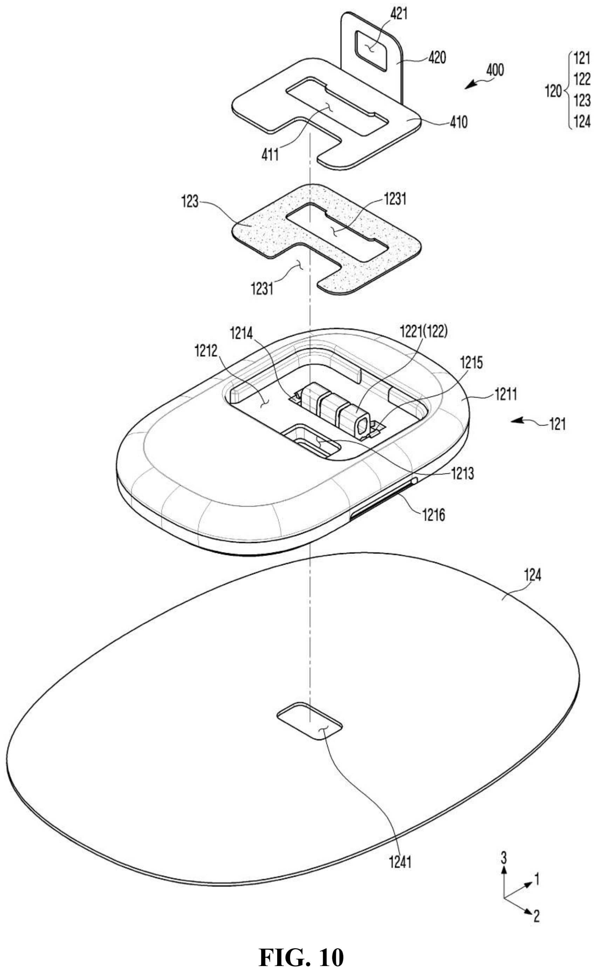

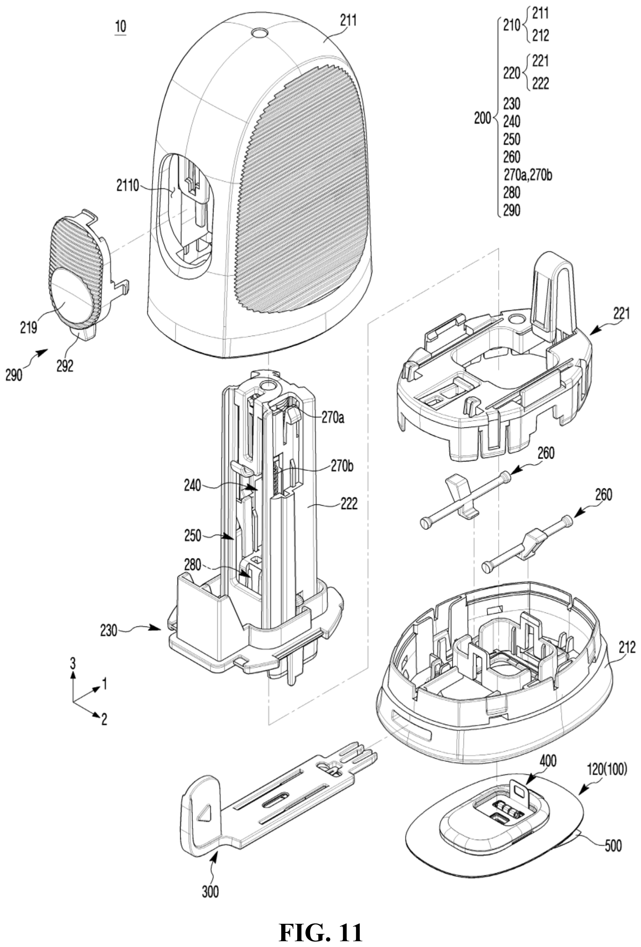

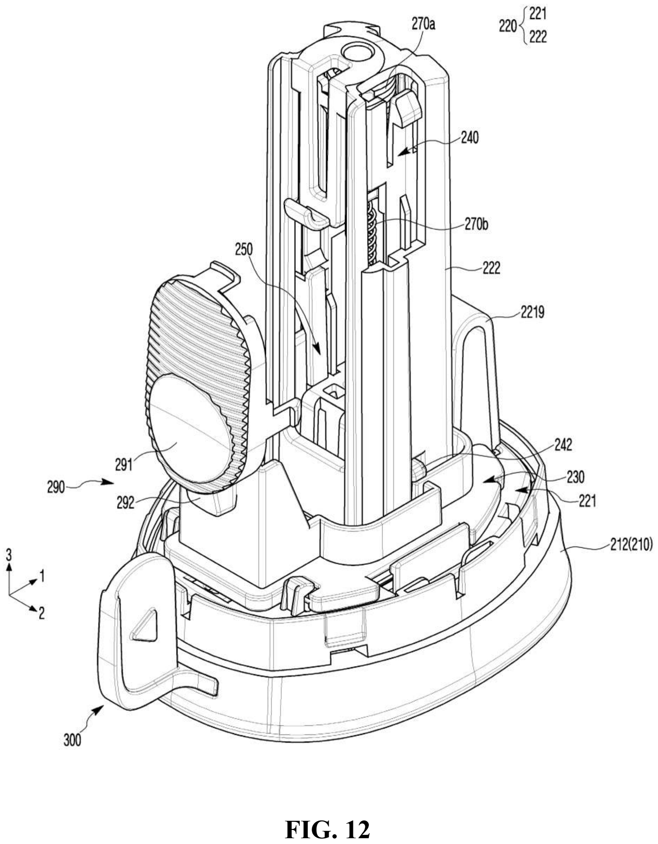

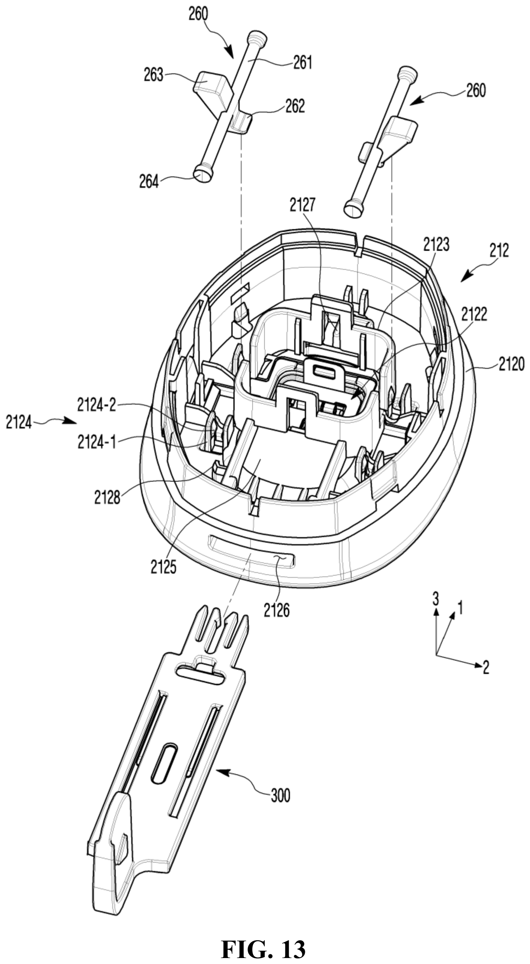

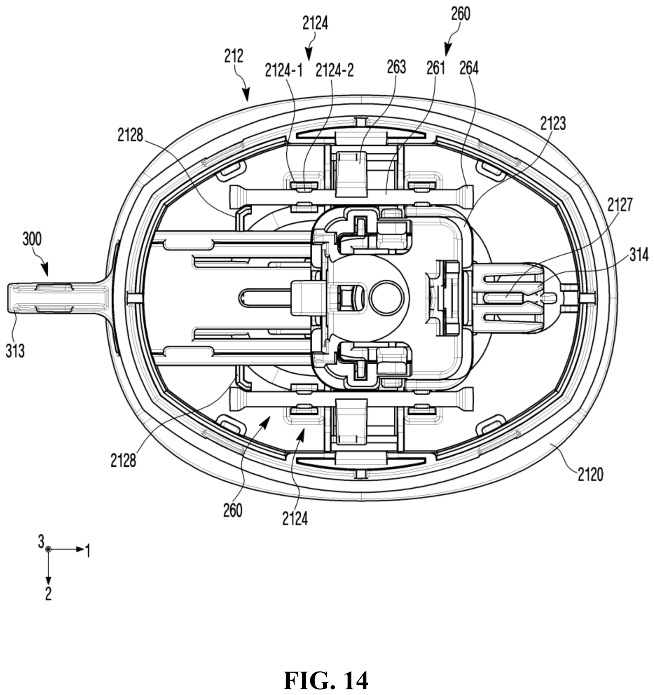

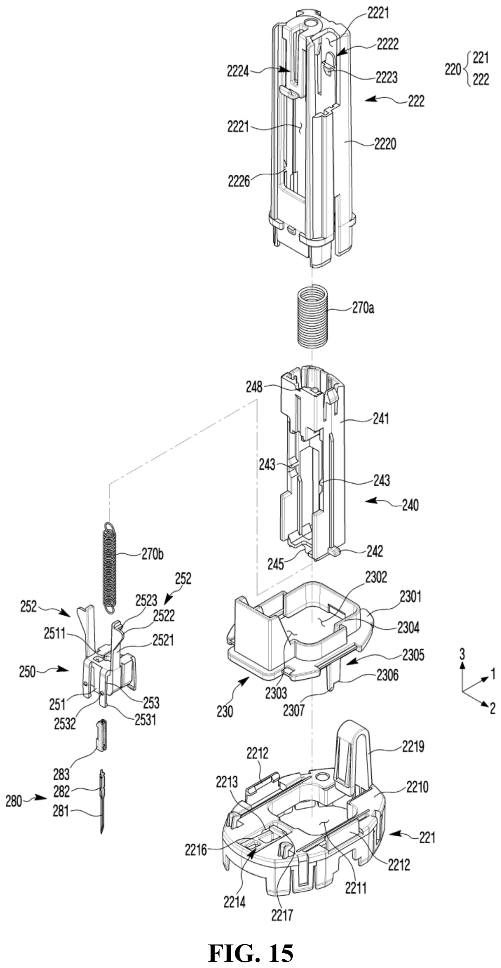

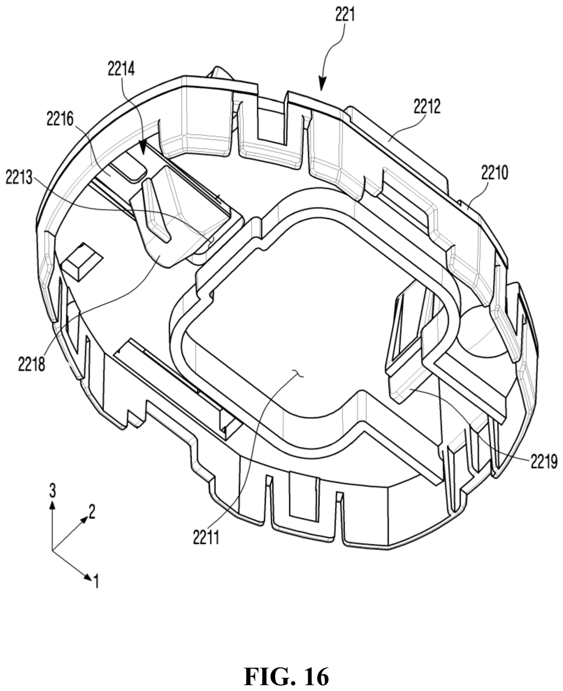

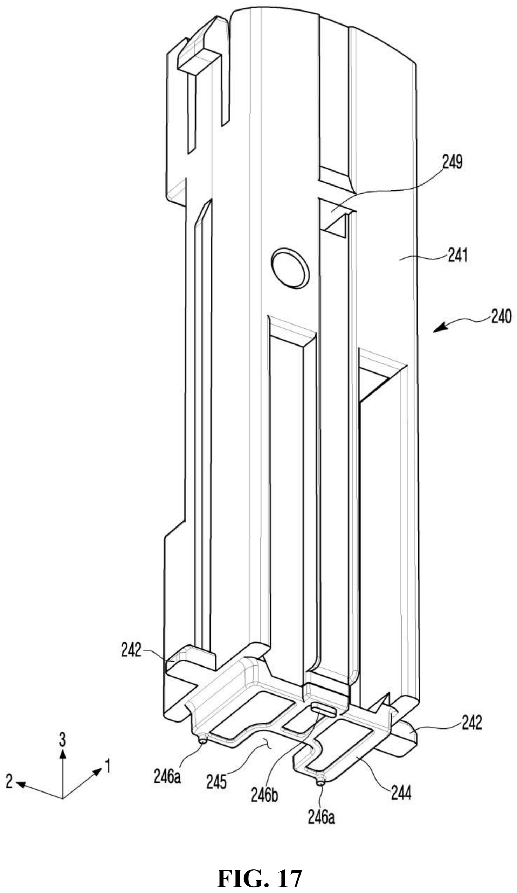

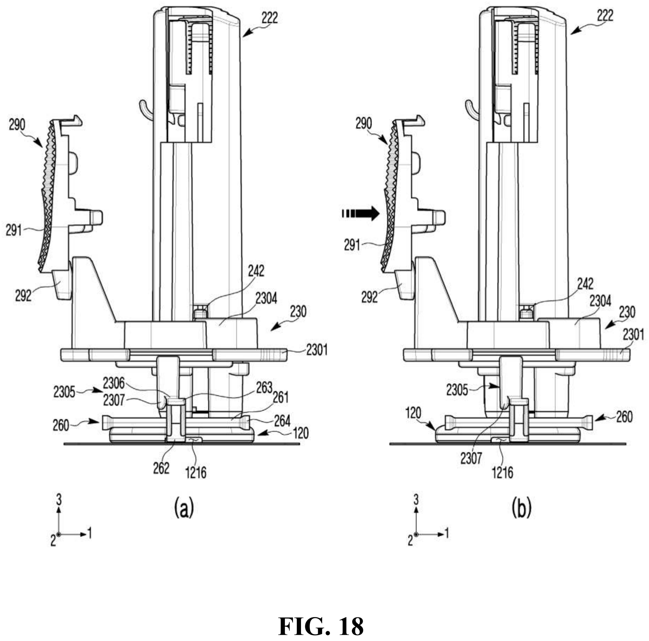

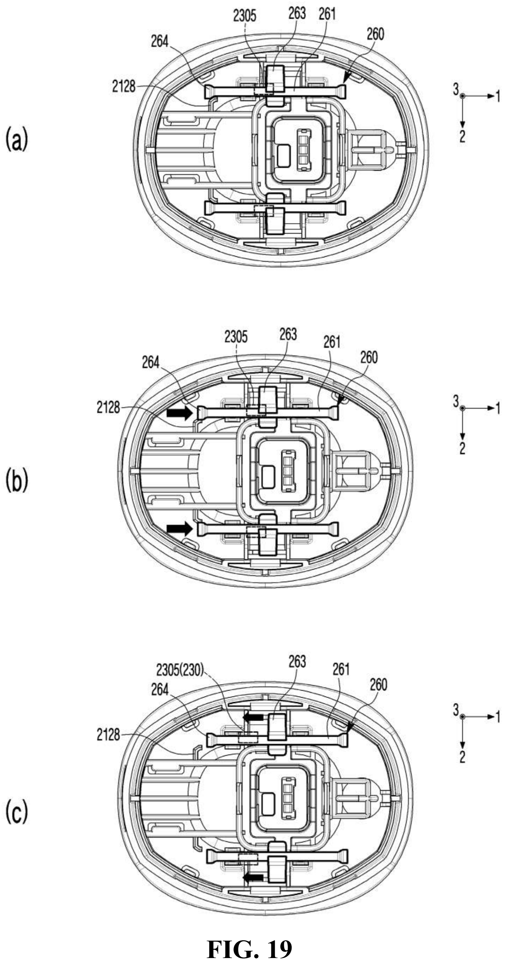

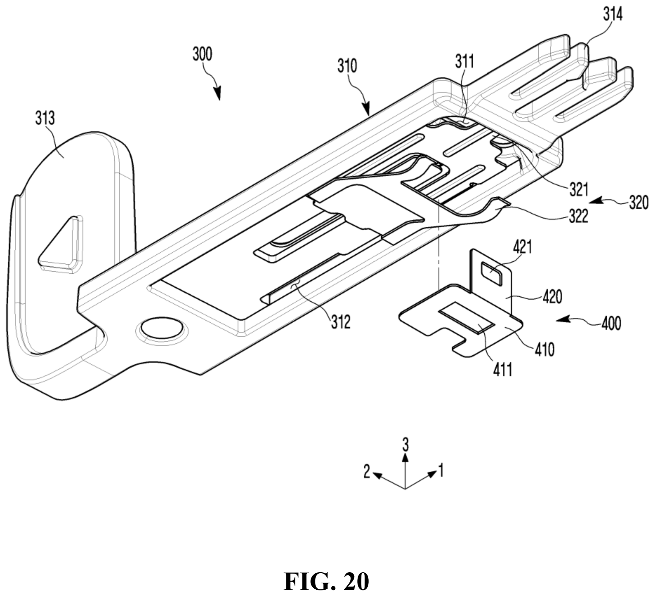

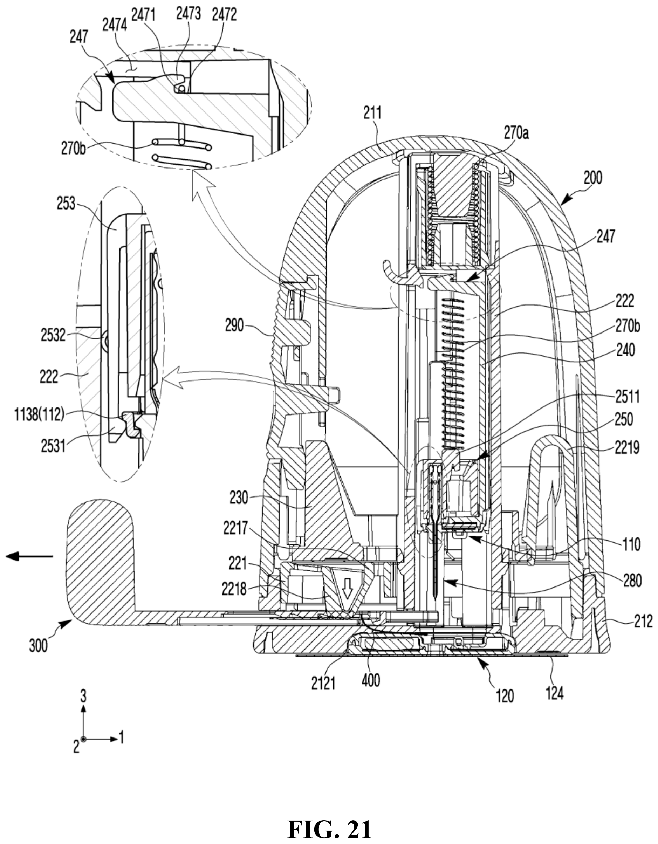

FIG. 1 is a perspective view schematically illustrating an applicator assembly according to an embodiment of the present disclosure. FIG. 2 is a diagram schematically illustrating a state in which a sensing device of an applicator assembly is attached to a user's skin according to an embodiment of the present disclosure. FIG. 3 is a diagram schematically illustrating a state of a sensor unit and a transmitter of a sensing device before being coupled according to an embodiment of the present disclosure. FIG. 4 is a diagram schematically illustrating a coupled state of a sensing device according to an embodiment of the present disclosure. FIG. 5 is a diagram schematically illustrating a cross-section along line III-III' of FIG. 4. FIG. 6 is a diagram schematically illustrating a cross-section along line IV-IV' of FIG. 4. FIGS. 7 and 8 are diagrams schematically illustrating an exploded view of a sensor unit, respectively. FIG. 9 is a diagram schematically illustrating a state in which a needle is coupled to a sensor unit. FIG. 10 is a diagram schematically illustrating a transmitter and a protective sheet. FIG. 11 is a diagram schematically illustrating an exploded view of an applicator assembly according to an embodiment of the present disclosure. FIG. 12 is a diagram schematically illustrating a coupled state of FIG. 11, excluding a top cover. FIG. 13 is a diagram schematically illustrating an exploded view of a base cover, a supporter, and a removing unit. FIG. 14 is a diagram illustrating a coupled state of a base cover, a supporter, and a removing unit as viewed from above. FIG. 15 is a diagram schematically illustrating an exploded view of an inner frame, a moving plate, a plunger, a carrier, a needle, a plunger spring, and a needle spring. FIG. 16 is a diagram schematically illustrating a middle frame as viewed from below. FIG. 17 is a diagram schematically illustrating a plunger as viewed from below. FIGS. 18A and 18B are diagrams schematically illustrating an operation of a moving plate and a supporter by pressing a release button. FIGS. 19A, 19B, and 19C are diagrams schematically illustrating an operation process of a supporter by a movement of a moving plate as viewed from above, respectively. FIG. 20 is a diagram schematically illustrating a removing unit and a protective sheet applied to the present embodiment. FIGS. 21 to 26 are diagrams schematically illustrating a manipulation method of an applicator assembly according to an embodiment of the present disclosure, wherein FIGS. 21, 22, and 24 are each diagrams schematically illustrating a cross-section along a line corresponding to line I-I' of FIG. 1, and FIGS. 23, 25, and 26 are each drawings schematically illustrating a cross-section along a line corresponding to line II-II' of FIG. 1.

Referring to FIGS. 1 and 2, the applicator assembly 10 is a mechanism for attaching and inserting a sensing device (100 of FIG. 2) that measures the user's biometric information into the user's skin.

Referring to FIGS. 1 and 11, the applicator assembly 10 according to an embodiment of the present disclosure includes a sensing device 100, an applicator 200, a removing unit 300, a protective sheet 400, and a release paper 500.

### 1. Sensing device

Referring to FIG. 2, a sensing device 100 may be attached and inserted into a user's body to measure the user's biometric information and communicate with an external terminal 5. For example, the sensing device 100 and the terminal 5 may be components of a continuous glucose monitoring system (CGMS). The sensing device 100 includes a sensor unit 110 and a transmitter 120. The sensor unit 110 and the transmitter 120 may be provided to the user in a form spaced apart from each other within an applicator 200 to be described later. When the user manipulates the applicator 200, the sensor unit 110 and the transmitter 120 may be coupled to each other to form the sensing device 100. The sensing device 100 may be detached from the applicator 200 and attached to the user's body. Hereinafter, the sensing device 100 is used to mean both a state in which the sensor unit 110 and the transmitter 120 are spaced apart from each other, and a state in which the sensor unit 110 and the transmitter 120 are coupled to each other.

The terminal 5 may receive biometric information-related data from the sensing device 100 and display it to the user. The terminal 5 may include, for example, a mobile phone, a laptop, a desktop, a dedicated medical device, or another type of computing device.

Meanwhile, in the present specification, it is assumed that the sensor unit 110 and the transmitter 120 are disposed to be spaced apart from each other within the applicator 200 and are coupled to each other by the user's manipulation of the applicator 200. However, this description does not exclude other types of sensing devices from the scope of the present disclosure. As another non-limiting example, the sensor unit 110 and the transmitter 120 may be disposed in a coupled form within the applicator 200, and while maintaining the coupled state, may be moved in the opposite direction to a third direction 3 by the user's manipulation of the applicator 200 to be attached to the user's skin, and may be detached from the applicator 200 while maintaining the coupled state.

In addition, in the following description, it is assumed that the first user who manipulates the applicator 200, the second user to whom the sensing device 100 is inserted and attached, and the third user who uses the terminal 5 are the same, but the scope of the present disclosure is not limited thereto. As another non-limiting example, at least one of the first to third users may be different from the others. As another non-limiting example, the first to third users may be different from each other.

### Sensor unit

The sensor unit 110 is inserted into the user's skin, at least in portion, to sense the user's biometric information. The sensor unit 110 may be disposed to be spaced apart from the transmitter 120 to be described later within the applicator 200 to be described later. The sensor unit 110 may be coupled with the transmitter 120 by the user's manipulation of the applicator 200 to form a sensing device 100.

Referring to FIGS. 3 to 9, the sensor unit 110 includes a transcutaneous sensor 111, a sensor unit housing 112, a sensor adhesive portion 114, and an adhesive pad 115.

### Transcutaneous sensor

The transcutaneous sensor 111 may be inserted into the user's skin, at least in portion, to sense biometric information. The transcutaneous sensor 111 includes a sensor body 1111, a connecting portion 1112, an intermediate portion 1113, an insertion portion 1116, and an extension portion 1117.

The sensor body 1111 may be formed in a plate shape overall. The sensor body 1111 may be disposed inside a sensor unit housing 112 to be described later. When the transcutaneous sensor 111 is disposed inside the sensor unit housing 112, one surface of the sensor body 1111 (based on the direction of FIG. 5, etc.) may be exposed to the outside of the sensor unit housing 112.

The connecting portion 1112 may extend from one side of the sensor body 1111 to connect the sensor body 1111 and the intermediate portion 1113 to be described later. The connecting portion 1112 may allow the sensor body 1111 and the intermediate portion 1113 to be placed on different planes. For example, as illustrated in the drawing, the connecting portion 1112 may have a shape that extends from one side of the sensor body 1111 in the opposite direction to the first direction 1 and then in the opposite direction to the third direction 3.

The intermediate portion 1113 may extend from the lower end portion of the connecting portion 1112 in the opposite direction to the second direction 2. The intermediate portion 1113 may connect the connecting portion 1112 and the insertion portion 1116. The intermediate portion 1113 may allow the axes of the connecting portion 1112 and the insertion portion 1116 along the third direction 3 to be spaced apart from each other in the second direction 2.

The insertion portion 1116 may be connected to an end portion of the intermediate portion 1113 along the opposite direction to the second direction 2. The insertion portion 1116 may be formed in a relatively thin and long probe shape so that it may be smoothly inserted into the user's skin.

The extension portion 1117 is formed in the sensor body 1111. For example, the extension portion 1117 may be formed in at least one of both end portions of the sensor body 1111 along the second direction 2. The extension portion 1117 may restrict movement of the sensor body 1111 within the sensor unit housing 112 to be described later.

For example, the transcutaneous sensor 111 may have a shape as shown in FIG. 7 or the like, by manufacturing the sensor body 1111, the connecting portion 1112, the intermediate portion 1113, the insertion portion 1116, and the extension portion 1117 in a flat shape and then bending the connecting portion 1112. Due to the bending deformation of the connecting portion 1112, the intermediate portion 1113 and the insertion portion 1116 may be disposed on different planes from the sensor body 1111, but the scope of the present disclosure is not limited thereto.

Meanwhile, in FIGS. 7 and 8, the intermediate portion 1113 and the insertion portion 1116 are disposed on the same plane, and the intermediate portion 1113 is disposed perpendicular to the sensor body 1111, but the angle between the intermediate portion 1113 and the sensor body 1111 or the angle between the insertion portion 1116 and the sensor body 1111 may be varied.

### Sensor unit housing

The sensor unit housing 112 forms the overall outer shape of the sensor unit 110. The sensor unit housing 112 may accommodate at least a portion of the transcutaneous sensor 111 therein. For example, the sensor body 1111, the connecting portion 1112, the intermediate portion 1113, and the extension portion 1117 of the transcutaneous sensor 111 are disposed inside the sensor unit housing 112, and at least a portion of the insertion portion 1116 of the transcutaneous sensor 111 may protrude from the lower portion of the sensor unit housing 112. The sensor unit housing 112 includes a housing base 1120 and a housing cap 1130.

The housing base 1120 may be coupled with a housing cap 1130 to be described later to form an accommodation space for accommodating at least a portion of the transcutaneous sensor 111 therein. The housing base 112 includes a housing base body 1121, a base penetration hole 1122, a base boss 1123, a boss penetration hole 1124, a base guide 1125, a base coupling groove 1126, a base protrusion 1127, a base leg 1128, and a base engaging portion 1129.

The housing base body 1121 may form the overall outer shape of the housing base 1120. The housing base body 1121 has one surface (the upper surface of the housing base body 1121 based on the direction of FIG. 7) and the other surface (the lower surface of the housing base body 1121 based on the direction of FIG. 7) facing in the third direction 3, and may be formed in a plate shape overall. The sensor body 1111 may be disposed on one surface of the housing base body 1121.

The base penetration hole 1122 may penetrate the housing base body 1121 in the third direction 3. The base penetration hole 1122 may expose one surface of the sensor body 1111 disposed inside the sensor unit housing 112 to the outside of the sensor unit housing 112. When coupling the sensor unit 110 and the transmitter 120, an electrical contact portion 1221 of the transmitter 120, which will be described later, may be physically and electrically connected to the sensor body 1111 through the base penetration hole 1122.

The base boss 1123 may be formed on the housing base body 1121. For example, the base boss 1123 may be formed on one side portion among the two side portions (one side portion and the other side portion) of the housing base body 1121 facing each other in the first direction 1. The base boss 1123 includes a first region 1123-1 formed to protrude on one surface of the housing base body 1121 and a second region 1123-1 formed to protrude on the other surface of the housing base body 1121. The first region 1123-1 of the base boss 1123 may be inserted into a cap penetration hole 1132 of the housing cap 1130 when the housing base 1120 and the housing cap 1130 are coupled. The second region 1123-2 of the base boss 1123 may be inserted into an insertion hole 1213 of the transmitter 120 when the sensor unit 110 and the transmitter 120 are coupled.

The boss penetration hole 1124 may penetrate the housing base body 1121 and the base boss 1123 in the third direction 3. At least a portion of each of the connection portion 1112, the intermediate portion 1113, and the insertion portion 1116 of the transcutaneous sensor 111 may be inserted into the boss penetration hole 1124. The insertion portion 1116 of the transcutaneous sensor 111 may be exposed to the outside of the sensor unit housing 112 through the boss penetration hole 1124. The needle 280 to be described later may penetrate the sensor unit housing 112 through the boss penetration hole 1124. A first portion formed in the first region 1123-1 of the base boss 1123 among the boss penetration hole 1124 may be opened in the second direction 2. A second portion formed in the housing base body 1121 among the boss penetration hole 1124 may communicate with the first portion and extend along the second direction 2 more than the first portion. Due to the first portion and the second portion, the intermediate portion 1113 extending in the second direction 2 may be inserted into the boss penetration hole 1124. A third portion formed in the second region 1123-2 of the base boss 1123 among the boss penetration hole 1124 may accommodate a portion of the insertion portion 1116 of the transcutaneous sensor 111.

The base guide 1125 may be formed to protrude on one surface of the housing base body 1121. The base guide 1125 may be formed on the edge side of one surface of the housing base body 1121. The inner wall of the base guide 1125 may define a region on one surface of the housing base body 1121 where the sensor body 1111 is disposed. A plurality of base guides 1125 may be formed to be spaced apart from each other on one surface of the housing base body 1121. The extension portion 1117 of the transcutaneous sensor 111 may be disposed in at least a portion of the space between the base guides 1125. When the sensor body 1111 is disposed on one surface of the housing base body 1121, the base guide 1125 may restrict movement of the sensor body 1111 along at least one of the first direction 1 (including the opposite direction to the first direction 1) and the second direction 2 (including the opposite direction to the second direction 2).

The base coupling groove 1126 may be formed on each of one side portion and the other side portion of the housing base body 1121. The base protrusion 1127 may be formed on the housing base body 1121 and the base boss 1123 so as to protrude into the base coupling groove 1126 when viewed from above. When the housing base 1120 and the housing cap 1130 are coupled, a cap protrusion 1135 of the housing cap 1130 is inserted into the base coupling groove 1126, and the base protrusion 1127 protruding to the base coupling groove 1126 may engage with the cap protrusion 1135 of the housing cap 1130. The base protrusion 1127 may include a lower end portion whose thickness (dimension along the first direction 1) increases toward the upper portion, and an upper end portion connected to the lower end portion and whose thickness decreases toward the upper portion. Since the thickness of the upper end portion of the base protrusion 1127 decreases toward the upper portion, when the housing base 1120 and the housing cap 1130 are coupled, the base protrusion 1127 may easily pass through the lower end portion of the cap protrusion 1135. Since the thickness of the lower end portion of the base protrusion 1127 increases toward the upper portion, after being engaged with the cap protrusion 1135, the base protrusion 1127 may restrict the movement of the cap protrusion 1135 along the third direction 3.

The base leg 1128 may be formed to protrude on the other surface of the housing base body 1121. When the sensor unit 110 and the transmitter 120 are coupled, at least a portion of the base leg 1128 may be inserted into the exposure hole 1214 of the transmitter 120 and coupled with the coupling protrusion 1215 of the transmitter 120. When the base leg 1128 and the coupling protrusion 1215 are coupled, movement of the sensor unit 110 with respect to the transmitter 120 may be restricted. The base leg 1128 includes leg portions 1128-1 each formed to protrude toward the lower portion on the other surface of the housing base body 1121 and formed to be spaced apart from each other, a connection portion 1128-2 that connects the lower end portions of the leg portions 1128-1 spaced apart from each other, and a fastening hole 1128H defined by the leg portions 1128-1 and the connection portion 1128-2. Referring to FIGS. 6 and 10, the connection portion 1128-2 may have a thickness of an upper end portion (meaning a dimension of the upper end portion of the connection portion 1128-2 along the second direction) that is thicker than a thickness of a lower end portion. For example, the thickness of the connection portion 1128-2 may decrease from the upper end portion toward the lower end portion. The connection portion 1128-2 may have an inner surface and an outer surface that face each other in the second direction 2. The inner surface of the connection portion 1128-2 may be substantially perpendicular to the other surface of the housing base body 1121. The outer surface of the connection portion 1128-2 may be inclined so as to become closer to the central portion of the housing base body 1121 toward the lower portion. According to this structure, when the sensor unit 110 and the transmitter 120 are coupled, the base leg 1128 may be easily inserted into the exposure hole 1214. In addition, when the sensor unit 110 and the transmitter 120 are coupled, the connection portion 1128-2 may easily pass through the coupling protrusion 1215 protruding from the inner wall of the exposure hole 1214, so that the base leg 1128 may easily dispose the coupling protrusion 1215 within the fastening hole 1128H. For example, two base legs 1128 may be formed facing each other in the second direction 2 around the base penetration hole 1122. As another example, a single or two or more multiple base legs 1128 may be formed around the base penetration hole 1122. The fastening hole 1128H may be communicated with the base penetration hole 1122, but the scope of the present disclosure is not limited thereto.

The base engaging portion 1129 may be formed in the housing base body 1121 and the base guide 1125. The base engaging portion 1129 may be a region defined by the upper surface of the housing base body 1121 and the outer wall of the base guide 1125. The base engaging portion 1129 may be formed in two corner regions spaced apart in the second direction 2 of one side portion of the housing base body 1121. When the housing cap 1130 and the housing base 1120 are coupled, at least a portion of the cap leg 1137 of the housing cap, which will be described later, may be accommodated in the base engaging portion 1129.

The housing cap 1130 may be coupled with the housing base 1120 to form an accommodation space that accommodates at least a portion of the transcutaneous sensor 111 therein. The housing cap 1130 includes a housing cap body 1131, a cap penetration hole 1132, a retention protrusion 1133, a sensor unit groove 1134, a cap protrusion 1135, a cap coupling groove 1136, a cap leg 1137, and a cap fence 1138.

The housing cap body 1131 may form the overall outer shape of the housing cap 1130. The housing cap body 1131 has one surface (the upper surface of the housing cap body 1131 based on the direction of FIG. 7) and the other surface (the lower surface of the housing cap body 1131 based on the direction of FIG. 7) facing in the third direction 3, and may be formed in a plate shape overall.

The cap penetration hole 1132 may penetrate the housing cap body 1131 in the third direction 3. For example, the cap penetration hole 1132 may be formed by penetrating the housing cap body 1131 at one side portion among the two side portions (one side portion and the other side portion) of the housing cap body 1131 facing each other in the first direction 1. When the housing base 1120 and the housing cap 1130 are coupled, the first region 1123-1 of the base boss 1123 may be inserted into the cap penetration hole 1132. Since at least a portion of the base boss 1123 is inserted into the cap penetration hole 1132, the boss penetration hole 1124 may be exposed to the outside of the sensor unit housing 112. Accordingly, the needle 280 outside the sensor unit housing 112 may be inserted into the sensor unit 110 through the boss penetration hole 1124.

The retention protrusion 1133 may be formed to protrude on the lower surface of the housing cap body 1131. When the housing base 1120 and the housing cap 1130 are coupled, at least a portion of the retention protrusion 1133 may be inserted into the boss penetration hole 1124 and come into contact with the intermediate portion 1113 of the transcutaneous sensor 111 disposed in the boss penetration hole 1124. Accordingly, the retention protrusion 1133 may restrict the movement of the transcutaneous sensor 111 along the third direction 3 within the sensor unit housing 112. In addition, when inserting the transcutaneous sensor 111 into the user's skin, the retention protrusion 1133 may press the intermediate portion 1113 to prevent the insertion portion 1116 from retracting.

The sensor unit groove 1134 may be formed on the upper surface of the housing cap body 1131. A pressing protrusion (246a of FIG. 17) of the plunger 240 which will be described later may be inserted into the sensor unit groove 1133. At least a portion of the cap leg 1137 which will be described later may be extended on the lower surface of the housing cap body 1131 corresponding to the formation location of the sensor unit groove 1134. The extended portion of the cap leg 1137 may protrude from the lower surface of the housing cap body 1131 to prevent the sensor unit groove 1134 from penetrating the housing cap body 1131 in the third direction 3.

The cap coupling groove 1136 may be formed on each of one side portion and the other side portion of the housing cap body 1131. The cap protrusion 1135 is formed on the housing cap body 1131 and may protrude to the cap coupling groove 1136 when viewed from above. When the housing base 1120 and the housing cap 1130 are coupled, the base protrusion 1127 of the housing base 1120 is inserted into the cap coupling groove 1136, and the cap protrusion 1135 protruding to the cap coupling groove 1136 may engage with the base protrusion 1127 of the housing base 1120. The cap protrusion 1135 may include lower end portion and upper end portion that have different thicknesses (dimensions along the first direction 1) and are connected to each other. The thickness of the lower end portion of the cap protrusion 1135 may decrease toward the lower portion. The thickness of the upper end portion of the cap protrusion 1135 may be substantially constant or may increase toward the lower portion. Since the thickness of the lower end portion of the cap protrusion 1135 decreases toward the lower portion, when the housing base 1120 and the housing cap 1130 are coupled, the cap protrusion 1135 may easily pass through the upper end portion of the base protrusion 1127. Since the thickness of the upper end portion of the cap protrusion 1135 is substantially constant or increases toward the lower portion, after engaging with the base protrusion 1127, the cap protrusion 1135 may restrict movement in the opposite direction to the third direction 3 of the base protrusion 1127. Meanwhile, the cap coupling groove 1136 formed on one side portion of the housing cap body 1131 may communicate with the cap penetration hole 1132. A guide protrusion (246b of FIG. 17) of the plunger 240 to be described later may be inserted into the cap coupling groove 1136 formed on the other side portion of the housing cap body 1131 to guide the location of the sensor unit **110** when assembling the plunger 240 and the sensor unit **110.**

The cap leg 1137 may be formed to protrude on the lower surface of the housing cap body 1131. The cap leg 1137 may be formed in two corner regions spaced apart in the second direction 2 of one side portion of the housing base body 1131. When the housing cap 1130 and the housing base 1120 are coupled, the cap leg 1137 may cover the side surface of the housing base 1120. At least a portion of the cap leg 1137 may extend to the lower surface of the housing cap body 1131 corresponding to the formation location of the sensor unit groove 1134. The extended portion of the cap leg 1137 may be accommodated in the base engaging portion 1129 of the housing base 1120 as described above.

The cap fence 1138 may be formed to protrude from an edge of the lower surface of the housing cap body 1131. The thickness (dimension along the first direction 1 or the second direction 2) of the cap fence 1138 may increase toward the upper portion. For example, the cap fence 1138 may have an inner wall substantially perpendicular to the lower surface of the housing cap body 1131, and an inclined surface connecting the lower end portion of the inner wall of the cap fence 1138 and the edge of the lower surface of the housing cap body 1131. When the housing cap 1130 and the housing base 1120 are coupled, the inner wall of the cap fence 1138 faces the outer wall of the base guide 1125, and at least a portion of the inclined surface of the cap fence 1138 may protrude further than the housing base 1120 when viewed from above. In addition, the protrusion distance of the portion of the cap fence 1138 that protrudes further than the housing base 1120 may decrease toward the lower portion. According to the structure of the cap fence 1138, a grip catching portion 2531 of a carrier 250 to be described later may be easily coupled with the sensor unit housing 112. In addition, the grip catching portion 2531 may be easily moved in the third direction 3 along the inclined surface of the cap fence 1138 to be disengaged from the sensor unit housing 112.

### Sensor adhesive portion

The sensor adhesive portion 114 may be disposed between the housing base 1120 and the sensor body 1111, and may couple the sensor body 1111 and the housing base 1120. The sensor adhesive portion 114 may be a material including a tape, paste, or resin having adhesive properties. For example, the sensor adhesive portion 114 may be formed of a double-sided tape having adhesive properties on both sides, and may be adhered to each of the sensor body 1111 and the housing base 1120, but the scope of the present disclosure is not limited thereto. A sensor adhesive portion opening 1140 may be formed in the central portion of the sensor adhesive portion 114. When the sensor unit 110 and the transmitter 120 are coupled, an electrical contact portion 1221, which will be described later, may be inserted into the sensor adhesive portion opening 1140. In other words, when the sensor unit 110 and the transmitter 120 are coupled, the electrical contact portion 1221 may contact the lower surface of the sensor body 1111 through the sensor adhesive portion opening 1140 and the base penetration hole 1122. The size and location of the sensor adhesive portion opening 1140 may be determined so that the sensor adhesive portion 1140 is not exposed through the base penetration hole 1122, but the scope of the present disclosure is not limited thereto. As another example, the size and location of the sensor adhesive portion opening 1140 may be determined so that at least a portion of the sensor adhesive portion 1140 is exposed through the base penetration hole 1122, provided that the electrical contact portion 1221 may be inserted into the sensor adhesive portion opening 1140. An adhesive portion groove 1141 may be formed at the edge of the sensor adhesive portion 114. The adhesive portion groove 1141 may expose the base protrusion 1127 of the housing base 1120 even when the sensor adhesive portion 114 is coupled to one surface of the housing base 1120.

### Adhesive pad

The adhesive pad 115 may be disposed between the housing base 1120 and the housing cap 1130 to couple the housing base 1120 and the housing cap 1130 to each other. For example, the adhesive pad 115 may be in contact with the upper surface of the base guide 1125 of the housing base 1120 and the lower surface of the housing cap body 1131 of the housing cap 1130. In addition, the adhesive pad 115 may be in contact with at least a portion of the upper surface of the sensor body 1111. The adhesive pad 115 may be a material including a tape, paste, or resin having adhesive properties. For example, the adhesive pad 115 may be formed of a double-sided tape having adhesive properties on both sides, and may be adhered to each of the housing base 1120 and the housing cap 1130, but the scope of the present disclosure is not limited thereto. An adhesive pad engaging portion 1150 may be formed at a corner portion spaced apart in the second direction 2 of one side portion among the two side portions (one side portion and the other side portion) facing each other in the first direction 1 of the adhesive pad 115. The adhesive pad engaging portion 1150 may expose an extended portion of the cap leg 1137 extending to the lower surface of the housing cap body 1131, even when the adhesive pad 115 is attached to the housing cap 1130. Therefore, when the housing cap 1130 and the housing base 1120 are coupled, the extended portion of the cap leg 1137 may be accommodated in the housing engaging portion 1129 of the housing base 1120 through the adhesive pad engaging portion 1150. A pad penetration groove 1151 may be formed in the central portion of one side portion of the adhesive pad 115. The pad penetration groove 1151 may expose the cap penetration hole 1132 and the retention protrusion 1133 even when the adhesive pad 115 is attached to the housing cap 1130. Therefore, when the housing cap 1130 and the housing base 1120 are coupled, the first region 1123-1 of the base boss 1123 may be inserted into the cap penetration hole 1132, and the retention protrusion 1133 may be inserted into the boss penetration hole 1124. A pad groove 1152 may be formed on the other side portion of the adhesive pad 115. The pad groove 1152 may expose the cap protrusion 1135 and the cap coupling groove 1136 formed on the other side portion of the housing cap body 1131 even when the adhesive pad 115 is attached to the housing cap 1130.

### Transmitter

The transmitter 120 may be coupled with the sensor unit 110 and attached to the user's skin, and be electrically connected to the sensor unit 110 to process biometric information measured by the sensor unit 110 and transmit the information to an external terminal (5 in FIG. 2).

Referring to FIGS. 3 to 6 and FIG. 10, the transmitter 120 includes a transmitter housing 121, an electronic component 122, an adhesive layer 123, and an adhesive portion 124.

### Transmitter housing

The transmitter housing 121 may form the overall outer shape of the transmitter 120. The transmitter housing 121 may be coupled with the sensor unit housing 112 by the user's manipulation of the applicator 200. The transmitter housing 121 includes a transmitter housing body 1211, a mounting portion 1212, an insertion hole 1213, an exposure hole 1214, a coupling protrusion 1215, and a housing groove 1216.

The transmitter housing body 1211 may form the overall outer shape of the transmitter housing 121. For example, the transmitter housing body 1211 may have a shape in which both end portions of two straight lines that are spaced apart from each other and parallel are connected by a curve (race track oval shape) when viewed from above. The transmitter housing body 1211 may have one surface (the upper surface based on the direction of FIG. 8) and the other surface (based on the direction of FIG. 8) that face each other in a third direction 3. The transmitter housing body 1211 may have an internal space that accommodates an electronic component 122 to be described later. The transmitter housing body 1211 may be formed by coupling two or more separate components. For example, the transmitter housing body 1211 may be formed by separately forming an upper housing that forms the upper surface of the transmitter housing body 1211 and a lower housing that forms the lower surface of the transmitter housing body 1211 and then coupling them. Meanwhile, in the following, in view of the fact that the transmitter housing body 1211 forms the overall outer shape of the transmitter housing 121, there may be cases where one surface of the transmitter housing body 1211 and one surface of the transmitter housing 121 are used interchangeably, and the other surface of the transmitter housing body 1211 and the other surface of the transmitter housing 121 are used interchangeably.

The mounting portion 1212 may be formed on the upper surface of the transmitter housing body 1211. The mounting portion 1212 may refer to a non-penetrating recessed portion formed on the upper surface of the transmitter housing body 1211. The bottom surface and the inner wall of the mounting portion 1212 may define an accommodation space of the transmitter housing 121 in which at least a portion of the sensor unit 110 is accommodated when the sensor unit 110 and the transmitter 120 are coupled.

The insertion hole 1213 is formed in the transmitter housing body 1211. Specifically, the insertion hole 1213 may have a form extended from the bottom surface of the mounting portion 1212 to the lower surface of the transmitter housing body 1211 in the opposite direction to the third direction 3. When the sensor unit 110 and the transmitter 120 are coupled, the second region 1123-2 of the base boss 1123 of the sensor unit 110 may be inserted into the insertion hole 1213. As described above, the insertion portion 1116 of the transcutaneous sensor 111 and the needle 280 may be disposed within the boss penetration hole 1124 penetrating the base boss 1123 and exposed to the outside (lower portion) of the sensor unit 110. Therefore, the insertion portion 1116 of the transcutaneous sensor 111 and the needle 280 may be inserted into the insertion hole 1213 together with the second region 1123-2 of the base boss 1123 when the sensor unit 110 and the transmitter 120 are coupled. In addition, the insertion portion 1116 of the transcutaneous sensor 111 and the needle 280 may be exposed to the outside (lower portion) of the transmitter housing 121 through the insertion hole 1213 when the sensor unit 110 and the transmitter 120 are coupled.

The exposure hole 1214 is formed in the transmitter housing body 1211 and exposes at least a portion of the electronic component 122 disposed inside the transmitter housing body 1211 to the outside of the transmitter housing body 1211. Specifically, the exposure hole 1214 is formed on the bottom surface of the mounting portion 1212 to be spaced apart from the insertion hole 1213 in the first direction 1, so that the electrical contact portion 1221 of the electronic component 122 disposed inside the transmitter housing body 1211 may be exposed to the outside of the transmitter housing body 1211. The electrical contact portion 1221 exposed to the outside through the exposure hole 1214 may come into contact with the sensor body 1111 when the sensor unit 110 and the transmitter 120 are coupled. When the sensor unit 110 and the transmitter 120 are coupled, at least a portion of the base leg 1128 of the sensor unit 110 may be inserted into the exposure hole 1214.

The coupling protrusion 1215 is formed on the transmitter housing body 1211. The coupling protrusion 1215 may be coupled with the base leg 1128 of the sensor unit 110 inserted into the exposure hole 1214 when the sensor unit 110 and the transmitter 120 are coupled. When the coupling protrusion 1215 and the base leg 1128 are coupled with each other, movement of the sensor unit 110 along the third direction 3 with respect to the transmitter 120 may be restricted. Referring to FIG. 6, the coupling protrusion 1215 includes a support portion 1215-1 formed on the bottom surface of the mounting portion 1212, and a protrusion portion 1215-2 that extends from the support portion 1215-1 to protrude toward the inner wall of the exposure hole 1214. The thickness of the protrusion portion 1215-2 (the dimension of the protrusion portion 1215-2 along the second direction 2) may increase toward the lower portion. According to this structure, when the sensor unit 110 and the transmitter 120 are coupled, the protrusion portion 1215-2 may be more easily inserted into the fastening hole 1128H through the connection portion 1128-2 of the base leg 1128. The lower surface of the protrusion portion 1215-2 may be substantially perpendicular to the inner wall of the exposure hole 1214. According to this structure, the protrusion portion 1215-2 may maintain the coupled state between the sensor unit 110 and the transmitter 120. In other words, when an external force is applied to the sensor unit 110 along the third direction 3 after the protrusion portion 1215-2 is disposed in the fastening hole 1128H, the lower surface of the protrusion portion 1215-2 is caught by the connection portion 1128-2, and the protrusion portion 1215-2 is prevented from coming out of the fastening hole 1128H. As a result, the coupling state between the sensor unit 110 and the transmitter 120 may be maintained. The number and formation locations of the coupling protrusions 1215 may correspond to the number and formation locations of the base legs 1128.

The housing groove 1216 is formed in the transmitter housing body 1211. A supporter 260 of the applicator 200 to be described later may be inserted into the housing groove 1216. When the supporter 260 is inserted into the housing groove 1216, the transmitter 120 and the applicator 200 may be maintained in a coupled state. When the supporter 260 is removed from the housing groove 1216, the transmitter 120 and the applicator 200 may be detached from each other. For example, the housing groove 1216 may be formed on at least one of the two side surfaces (one side surface and the other side surface) of the transmitter housing body 1211 facing the second direction 2. For example, at least one or more housing grooves 1216 may be formed on one side surface of the transmitter housing body 1211 and at least one or more housing grooves 1216 may be formed on the other side surface of the transmitter housing body 1211. Meanwhile, if the transmitter housing body 1211 is formed by coupling two or more separate components, the housing groove 1216 may be formed only in some of the multiple components that constitute the transmitter housing body 1211, and may not be formed in the remaining components. For example, if the transmitter housing body 1211 is formed by coupling an upper housing and a lower housing, the housing groove 1216 may be formed only in the lower housing of the transmitter housing body 1211, and may not be formed in the upper housing of the transmitter housing body 1211, but the scope of the present disclosure is not limited thereto.

### Electronic component

The electronic component 122 is disposed within the transmitter housing body 1211 of the transmitter housing 121. When the sensor unit 110 and the transmitter 120 are coupled, the electronic component 122 is connected to the transcutaneous sensor 111. The electronic component 122 may generate biometric information-related data at multiple different points in time from the user's biometric information sensed by the transcutaneous sensor 111, and wirelessly transmit the biometric information-related data to a terminal 5 outside the sensing device 100. Here, the biometric information and biometric information-related data may be related to the body's glucose concentration, but the scope of the present disclosure is not limited thereto. The electronic component 122 includes a substrate 1220, an electrical contact portion 1221 in contact with the transcutaneous sensor 111, a battery 1222, and various chips. The substrate 1220 may support the electrical contact portion 1211, the battery 1222, and various chips. A circuit pattern for electrically connecting at least two or more of the electrical contact portion 1211, the battery 1222, and various chips may be formed on the substrate 1220. The electrical contact portion 1221 may be exposed through the exposure hole 1214 of the transmitter housing 121. When the sensor unit 110 and the transmitter 120 are coupled, the electrical contact portion 1221 may come into contact with the sensor body 1111 by passing through the base penetration hole 1122 and the sensor adhesive portion opening 1140. The electrical contact portion 1221 may be formed in a form that may be elastically deformed to ensure stable contact with the sensor body 1111. The electrical contact portion 1221 may be electrically connected to the substrate 1220. The chip may be disposed on the substrate 1220. The chip may include a processor chip for processing a signal and a communication chip for communicating with an external terminal (5 in FIG. 2).

### Adhesive layer

The adhesive layer 123 may be disposed on one surface of the transmitter housing 121 to couple the sensor unit housing 112 and the transmitter housing 121. For example, the adhesive layer 123 may be disposed on the bottom surface of the mounting portion 1212. The adhesive layer 123 may seal between the sensor unit housing 112 and the transmitter housing 121 to prevent moisture or foreign substances from entering the electrical contact portion 1221. An adhesive layer hole 1231 may be formed in the adhesive layer 123. The adhesive layer hole 1231 may expose at least a portion of the insertion hole 1213 of the transmitter housing 121. At least a portion of each of the needle 280 and the insertion portion 1116 of the transcutaneous sensor 111 may pass through the adhesive layer hole 1231 and the insertion hole 1213 of the transmitter housing 121. In addition, the adhesive layer hole 1231 may expose at least a portion of the electrical contact portion 1221. The adhesive layer 123 may be a material including a tape, paste, or resin having adhesive properties. For example, the adhesive layer 123 may be formed of a double-sided tape having adhesive properties on both sides, but the present disclosure is not limited thereto.

Based on a state before the sensor unit 110 and the transmitter 120 are coupled, one surface of the adhesive layer 123 may be coupled to the bottom surface of the mounting portion 1212, and the other surface of the adhesive layer 123 may be coupled to a protective sheet 400 to be described later. The protective sheet 400 may be detached from the other surface of the adhesive layer 123 when removing the removing unit 300 from the applicator 200. When the other surface of the adhesive layer 123 is exposed to the outside due to the removal of the protective sheet 400, the user may manipulate the applicator 200 to move the sensor unit 110 to the lower portion, thereby coupling the sensor unit housing 112 to the other surface of the adhesive layer 123.

Meanwhile, in the above, it has been described that the adhesive layer 123 is provided to the user in a state of being coupled to the transmitter housing 121, but the scope of the present disclosure is not limited thereto. As another example, the adhesive layer 123 may be provided to the user in a form coupled to the sensor unit housing 112 rather than the transmitter housing 121. As yet another example, the adhesive layer 123 may be provided to the user in a form spaced apart from each of the sensor unit housing 112 and the transmitter housing 121. In this example, when the user manipulates the applicator 200, the sensor unit 110 may be sequentially coupled with the adhesive layer 123 and the transmitter 120 while moving along the lower portion direction within the applicator 200 (in the opposite direction to the third direction 3).

### Adhesive portion

The adhesive portion 124 may attach the transmitter housing 121 to the user's skin. The adhesive portion 124 may be disposed on the other surface of the transmitter housing 121. The adhesive portion 124 has an upper surface that contacts the other surface of the transmitter housing 121 and a lower surface facing the upper surface. A release paper 500 to be described later may be attached to the lower surface of the adhesive portion 124 to prevent external exposure of the lower surface of the adhesive portion 124. When the user removes the release paper 500, the lower surface of the adhesive portion 124 is exposed to the outside, and since the lower surface of the adhesive portion 124 has adhesive properties, it may be attached to the user's skin. The adhesive portion 124 may be a material including a tape, paste, or resin having adhesive properties, and may be formed of a double-sided tape having adhesive properties on both sides, but the present disclosure is not limited thereto. An adhesive portion opening 1241 may be formed in the central portion of the adhesive portion 124 through which the insertion portion 1116 of the transcutaneous sensor 111 and the needle 280 may pass. As another example, the adhesive portion 124 may include an upper member coupled to the other surface of the transmitter housing 121, and a lower member coupled to the lower surface of the upper member. The upper member may be adhered to the other surface of the transmitter housing 121, so that it may not be exposed to the outside of the transmitter housing 121 when viewed from the upper portion of the transmitter housing 121. The lower member may be adhered to the lower surface of the upper member, so that it may be exposed to the outside of the transmitter housing 121 when viewed from the upper portion of the transmitter housing 121. The upper member may have adhesive properties on both the upper surface that contacts the other surface of the transmitter housing 121 and the lower surface that contacts the lower member. The lower member may not have adhesive properties on the upper surface that contacts the upper member, and may have adhesive properties only on the lower surface. The release paper 500 to be described later may be attached to the lower surface of the lower member. In this example, the above-described adhesive portion opening 1241 may be formed in a form that penetrates both the upper member and the lower member.

### 2. Protective sheet

The protective sheet 400 is disposed inside the applicator 200 in a state being coupled to the other surface of the adhesive layer 123. By the user removing the removing unit 300 to be described later from the applicator 200, the protective sheet 400 may be removed from the applicator 200 together with the removing unit 300, thereby exposing the other surface of the adhesive layer 123. Thereafter, by the user manipulating a release button 290 of the applicator 200 to be described later, the sensor unit 110 may be moved in the opposite direction to the third direction 3 to be coupled to the transmitter 120. The protective sheet 400 is formed of a releasable material and may be easily removed from the adhesive layer 123.

Referring to FIGS. 10 and 20, the protective sheet 400 includes a protective portion 410 and a wing portion 420.

The protective portion 410 may cover the other surface of the adhesive layer 123 to prevent external exposure of the adhesive layer 123. A protective sheet opening 411 may be formed in the protective portion 410. The protective sheet opening 411 may expose at least a portion of the adhesive layer hole 1231 of the adhesive layer 123. One surface of the protective portion 410 that comes into contact with the other surface of the adhesive layer 123 may include a release material.

The wing portion 420 is connected to the protective portion 410. In a state where the protective sheet 400 is coupled to the adhesive layer 123, the wing portion 420 may be supported by the inner wall of the mounting portion 1212 of the transmitter 120 and have a shape that extends in the third direction 3 from the protective portion 410. The removing unit 300 to be described later may be coupled to the wing portion 420. To this end, a protective sheet hole 421 for coupling with the removing unit 300 may be formed in the wing portion 420.

### 3. Applicator

The applicator 200 may cover at least a portion of each of the sensor unit 110 and the transmitter 120. The applicator 200 may couple the sensor unit 110 and the transmitter 120 to each other to form a sensing device 100. Thereafter, the applicator 200 may be detached from the sensing device 100 by a user. The applicator 200 may be detachably coupled to each of the removing unit 300, the protective sheet 400, and the release paper 500.

Referring to FIG. 1 and FIGS. 11 to 19, the applicator 200 includes a main frame 210, an inner frame 220, a moving plate 230, a plunger (240), a carrier 250, a supporter 260, a plunger spring 270a, a needle spring 270b, a needle 280, and a release button 290.

### Main frame

The main frame 210 may form the overall appearance of the applicator 200. The main frame 210 may cover at least a portion of each of the sensor unit 110 which is disposed inside, transmitter 120 and detailed configuration of the applicator 200 to be described later.

Referring to FIGS. 11 to 14, FIGS. 18 and 19, the main frame 210 includes a top cover 211 and a base cover 212.

The top cover 211 is coupled to the upper portion of the base cover 212 and may cover, together with the base cover 212, at least a portion of each of the sensor unit 110, the transmitter 120, and the detailed components of the applicator 200 to be described later. A top cover opening 2110 is formed in the top cover 211 to which the release button 290 to be described later is coupled.

The base cover 212 is coupled to the lower portion of the top cover 211 and may cover, together with the top cover 211, at least a portion of each of the sensor unit 110, the transmitter 120, and the detailed components of the applicator 200 to be described later. The base cover 212 is coupled to the inner frame 220 to be described later and may support the inner frame 220. The base cover 212 includes a base cover body 2120, a base recess 2121, a base cover opening 2122, a fence portion 2123, a support portion 2124, a base stage 2125, an entrance 2126, an engaging protrusion 2127, and a frame retention protrusion 2128.

The base cover body 2120 forms the overall outer shape of the base cover 212. The base cover body 2120 may have an upper surface and a lower surface facing each other in the third direction 3, and a side surface forming an internal space with an upper portion open.

The base recess 2121 (see FIG. 21) is formed on the lower surface of the base cover body 2120. The base recess 2121 may detachably accommodate the transmitter 120.

The base cover opening 2122 is formed by penetrating the base cover body 2120 in the third direction 3. The base cover opening 2122 may be in communication with the base recess 2121. When the user manipulates the applicator 200, the sensor unit 110 may pass through the base cover opening 2122 and be coupled with the transmitter 120 accommodated in the base recess 2121.

The fence portion 2123 may be disposed around the base cover opening 2122 on the upper surface of the base cover body 2120. The fence portion 2123 may be coupled with a column member 222 of the inner frame 220 to be described later to support the inner frame 220. At least a portion of the column member 222 may be inserted into the internal space formed by the fence portion 2123. A fence catching portion for coupling with the column member 222 may be formed on the upper portion of each of the two side surfaces facing in the first direction 1 of the fence portion 2123. A hole that engages with the column member 222 may be formed in the fence catching portion. A fence slit through which the removing unit 300 passes may be formed on the lower portion of each of the two side surfaces of the fence portion 2123. The fence slit may guide movement of the removing unit 300 in the opposite direction to the first direction 1 when a user removes the removing unit 300 from the applicator 200. The fence slit may be communicated with the hole of the fence catching portion.

The support portion 2124 may be disposed on the outer side of the fence portion 2123 in the second direction 2 and the opposite direction to the second direction 2 on the upper surface of the base cover body 2120, so as to rotatably support the supporter 260 to be described later. The support portion 2124 includes an elastic support 2124-1 that is formed to protrude in the third direction 3 on the upper surface of the base cover body 2120 and is elastically deformable in the second direction 2 (including the opposite direction to the second direction 2), and a support protrusion 2124-2 that protrudes from the surface of the elastic support 2124-1 toward the fence portion 2123. The support 2124-1 allows movement of the supporter 260 along the first direction 1, but may restrict movement of the supporter 260 along the second direction 2 (including the opposite direction to the second direction 2). The support protrusion 2124-2 may restrict movement of the supporter 260 along the third direction 3. Meanwhile, the support portion 2124 may be modified in various ways as long as it has a form that allows movement of the supporter 260 along the first direction 1, while restricting movement of the supporter 260 along the second direction 2 (including the opposite direction to the second direction 2) and the third direction 3.

The base stage 2125 is formed on the upper surface of the base cover body 2120 and supports the removing unit 300. The base stage 2125 may support the removing unit 300 so that the removing unit 300 may move linearly stably. The base stage 2125 may be a region that protrudes toward the inside of the base cover body 2120 due to the formation of a base recess 2121 on the lower surface of the base cover body 2120.

The entrance 2126 is formed on the side surface of the base cover body 2120, so that at least a portion of the removing unit 300 may be accommodated into the internal space of the base cover body 2120 through the entrance 2126. The entrance 2126, together with the base stage 2125 and the fence slit of the fence portion 2123, may guide the movement of the removing unit 300 in the base cover body 2120. The engaging protrusion 2127 may be formed on the upper surface of the base cover body 2120 and be engaged with the end portion of the removing unit 300.

A pair of frame retention protrusions 2128 may be provided to correspond to a pair of supporters 260 respectively disposed on both sides facing in the second direction 2 of the base cover opening 2122. The frame retention protrusion 2128 may be elastically deformable. The frame retention protrusion 2128 may allow movement of the supporter 264 along the first direction 1, and after movement of the supporter 264 along the first direction 1, may restrict movement of the supporter 264 along the opposite direction to the first direction 1.

### Release button

The release button 290 is coupled to the top cover opening 2110 of the top cover 211, and may move in the first direction 1 when the user presses the release button 290 in the first direction 1. When the moving plate 230 moves in the first direction 1 by the movement of the release button 290 along the first direction 1, movement in the opposite direction to the third direction 3 of each of the sensor unit 110, the plunger 240 and the carrier 250 to be described later may be allowed. When the user's pressure along the first direction 1 is released, the release button 290 may be moved in the opposite direction to the first direction 1.

Referring to FIGS. 11, 12, and 18, the release button 290 includes a button portion 291 and a button protrusion 292.

The button portion 291 may be disposed in the top cover opening 2110 of the main frame 210 and be exposed to the outside of the main frame 210. A user may press the release button 290 by pressing the button portion 291. The button protrusion 292 may be formed to protrude on one side of the button 510. The button protrusion 292 may come into contact with a stopper portion 231 inside the main frame 210. The button protrusion 292 may transmit an external force along the first direction 1 applied to the button portion 291 to the moving plate 230 and transmit an external force along the opposite direction to the first direction 1 applied to the moving plate 230 to the button portion 291.

### Inner frame

The inner frame 220 may be supported by being coupled to the base cover 212. The inner frame 220 may accommodate the sensor unit 110, the plunger 240 to be described later, the carrier 250 to be described later, and the needle 280 to be described later, and may form an internal space in which these components move in the opposite direction to the third direction 3 and in the third direction 3. The inner frame 220 may support the moving plate 230 to be described later.

Referring to FIGS. 11, 12, and 15, the inner frame 220 includes a middle frame 221 and the column member 222.

### Middle frame

The middle frame 221 may be coupled to the base cover 212. The middle frame 221 may support the column member 222 to be described later that penetrates the middle frame 221. The middle frame 221 may support the moving plate 230 coupled to the middle frame 221, which will be described later, and guide movement of the moving plate 230 along the first direction 1 (including the opposite direction to the first direction 1).

Referring to FIGS. 11, 15, and 16, the middle frame 221 includes a middle frame body 2210, a middle frame opening 2211, a guide protrusion 2212, a penetration hole 2213, a locking unit 2214, and an elastic pressing portion 2219.

The middle frame body 2210 may be inserted into the internal space formed by the side surface of a base cover body 2120 and coupled to the base cover 212. The upper surface of the middle frame body 2210 may form a stage on which the moving plate 230 to be described later moves along the first direction 1 (including the opposite direction to the first direction 1).

The middle frame opening 2211 may penetrate the central portion of the middle frame body 2210. When the middle frame 221 and the base cover 212 are coupled, the fence portion 2123 of the base cover 212 may be exposed through the middle frame opening 2211. The column member 222 to be described later may be inserted into the middle frame opening 2211 and coupled with the fence portion 2123 exposed through the middle frame opening 2211.

The guide protrusions 2212 may be disposed on both sides of the middle frame body 2210 facing each other in the second direction 2. The guide protrusion 2212 may protrude on the upper surface of the middle frame body 2210. The guide protrusion 2212 may guide the movement of the moving plate 230, which will be described later, in the first direction 1 (including the opposite direction to the first direction 1) on the upper surface of the middle frame body 2210. In other words, the guide protrusion 2212 may guide the moving plate 230 to move from the deactivation position to the activation position. Here, the deactivation position may mean a position at which the moving plate 230 blocks the movement of the sensor unit 110 and the plunger 240 in the opposite direction to the third direction 3. The activation position may mean a position at which the movement of the sensor unit 110 and the plunger 240 in the opposite direction to the third direction 3 is enabled as the moving plate 230 moves in the first direction 1.

The penetration hole 2213 may penetrate the middle frame body 2210 so as to be spaced apart from the middle frame opening 2211. The locking unit 2214 is disposed within the penetration hole 2213. The locking unit 2214 may engage with the moving plate 230 to prevent movement of the moving plate 230 along the first direction 1, and may be disengaged from the moving plate 230 to allow movement of the moving plate 230 along the first direction 1. The locking unit 2214 includes a body portion 2216 disposed within the penetration hole 2213, a hook portion 2217 disposed at the end portion of the body portion 2216, and a leg portion 2218 disposed on the lower surface of the body portion 2216. The body portion 2216 may have one end as a fixed end connected to the inner wall of the penetration hole 2213 and the other end as a free end spaced from the inner wall of the penetration hole 2213. Accordingly, the body portion 2216 may be elastically deformed in the third direction 3 (including the opposite direction to the third direction 3). The hook portion 2217 may be formed to protrude on the upper surface of the other end of the body portion 2216. The hook portion 2217 may be engaged with or disengaged from the moving plate 230 to be described later depending on whether the body portion 2216 is elastically deformed. If the removing unit 300 is not removed from the applicator 200, the leg portion 2218 of the locking unit 2214 may be in contact with the removing body 310, and elastic deformation of the locking unit 2214 in the third direction 3 (including the opposite direction to the third direction 3) may not be allowed. In this case, even if an external force is applied to the moving plate 230 in the first direction 1 by the user, the locking unit 2214 may remain in engagement with the moving plate 230, and the moving plate 230 may not move in the first direction 1. If the removing body 310 is removed from the applicator 200, elastic deformation of the locking unit 2214 in the third direction 3 (including the opposite direction to the third direction 3) may be allowed. In this case, when an external force is applied to the moving plate 230 in the first direction 1 by the user, the engagement between the locking unit 2214 and the moving plate 230 is released, and the moving plate 230 may be moved in the first direction 1.

The elastic pressing portion 2219 may be formed on the upper surface of the middle frame body 2210. The elastic pressing portion 2219 may elastically support the moving plate 230 so that the moving plate 230 does not easily move from the deactivation position to the activation position. **In** other words, the elastic pressing portion 2219 may generate a resistance force that resists the external force along the first direction 1 generated in the moving plate 230 as the user presses the release button 290 in the first direction 1. Therefore, the elastic pressing portion 2219 may cause the moving plate 230 to move along the first direction 1 only when the user applies a force greater than a certain level to the release button 290. **In** addition, the elastic pressing portion 2219 may press the moving plate 230 in the opposite direction to the first direction 1 when the user no longer presses the release button 290 after the moving plate 230 has moved to the activation position. Accordingly, the moving plate 230 may move in the opposite direction to the first direction 1 from the activation position.

### Column member

The column member 222 may be supported by being coupled to the base cover 212 by penetrating the middle frame 221. The column member 222 may accommodate the sensor unit **110,** the plunger 240 to be described later, the carrier 250 to be described later, and the needle 280 to be described later, and may form an internal space in which these components move in the opposite direction to the third direction 3 and in the third direction 3.

Referring to FIGS. **11****,** 15, and 18, the column member 222 includes a column member body 2220, a column member opening 2221, a clamping portion 2222, a blocking portion 2224, and a release portion 2226.

The column member body 2220 may form the overall outer shape of the column member 222. The column member body 2220 may have an overall hexahedral shape. The column member body 2220 may accommodate the sensor unit 110, the plunger 240 to be described later, the carrier 250 to be described later, and the needle 280 to be described later inside. The lower end of the column member body 2220 may be open. When the column member 222 is coupled to the base cover 212, the internal space of the column member body 2220 may be communicated with the base cover opening 2122 of the base cover 212. According to this structure, the sensor unit 110 disposed inside the column member body 2220 may be moved to the mounting portion 1212 of the transmitter 120 through the lower end of the column member body 2220 by the user's manipulation of the applicator 200. The column member opening 2221 may be formed on each of both side surfaces (one side surface and the other side surface) of the column member body 2220 facing in the first direction 1.

The clamping portion 2222 may be disposed on the column member opening 2221 formed on one side surface of the column member body 2220 (meaning the rear surface among the two surfaces of the column member body 2220 facing in the first direction 1 shown in FIG. 15). The clamping portion 2222 may restrict movement of the plunger 240 to be described later along the opposite direction to the third direction 3 with respect to the column member body 2220. The clamping portion 2222 may have a lower end as a fixed end connected to the inner wall of the column member opening 2221, and an upper end as a free end spaced apart from the inner wall of the column member opening 2221. Accordingly, the clamping portion 2222 may be elastically deformed in the first direction 1 (including the opposite direction to the first direction 1). The clamping portion 2222 has a clamping portion detent 2223 that may be engaged with one side of the plunger 240 to be described later. When the clamping portion detent 2223 and a plunger latch (249 of FIG. 17) of the plunger to be described later are engaged with each other, movement of the plunger 240 in the opposite direction to the third direction 3 with respect to the column member 222 may be restricted. When the inner frame 220 is coupled to the base cover 212 while the clamping portion detent 2223 and the plunger latch 249 are engaged with each other, movement of the plunger 240 due to carelessness of the operator may be prevented during the assembling process of the applicator 200. The engagement between the clamping portion detent 2223 and the plunger latch 249 may be released by the operator when the coupling process between the inner frame 220 and the base cover 212 is completed.

The blocking portion 2224 may be disposed on the column member opening 2221 formed on the other side surface of the column member body 2220 (meaning the front surface among the two surfaces of the column member body 2220 facing in the first direction 1 shown in FIG. 19). The blocking portion 2224 may have an upper end as a fixed end connected to the inner wall of the column member opening 2221, and a lower end as a free end spaced apart from the inner wall of the column member opening 2221. Accordingly, the blocking portion 2224 may be elastically deformed in the opposite direction to the first direction 1 to allow movement of the plunger 240 when the plunger 240, which will be described later, moves in the opposite direction to the third direction 3 with respect to the column member body 2220. When the movement of the plunger 240 in the opposite direction to the third direction 3 with respect to the column member body 2220 is completed, the blocking portion 2224 may engage with a plunger coupling groove 248 of the plunger 240 to fix the plunger 240 to the corresponding location. In other words, the blocking portion 2224 may prevent the plunger 240, which has completed its movement, from moving to the initial location, thereby preventing reuse of the applicator 200.

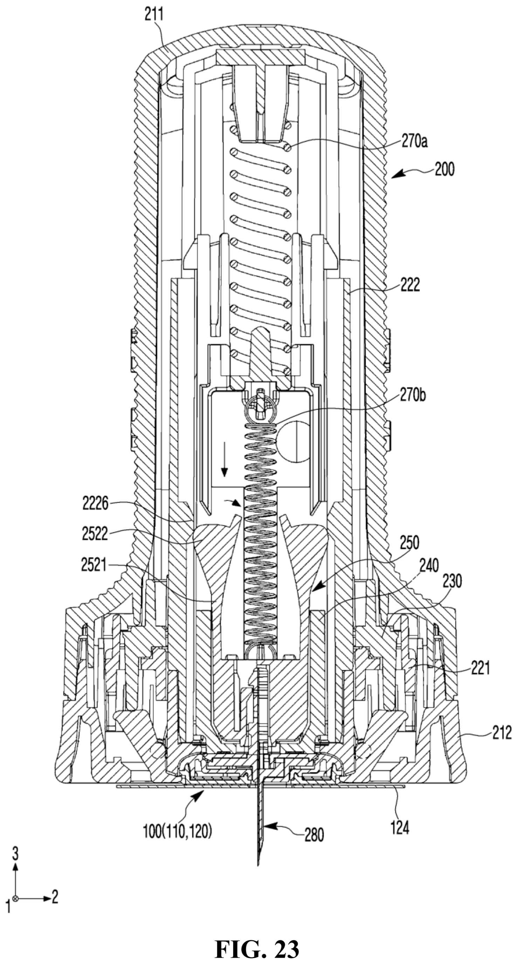

The release portion 2226 may be disposed on the inner surface of the column member body 2220. Specifically, the release portion 2226 may be disposed to protrude on each of two inner surfaces facing each other in the second direction 2 among the four inner surfaces of the column member body 2220. The release portion 2226 may contact a carrier wing 252 of the carrier 250 and pressurize the carrier wing 252 toward the inner side during the process in which the carrier 250, which will be described later, moves in the opposite direction to the third direction 3 while being engaged with the plunger 240 (see FIG. 23). When the release portion 2226 presses the carrier wing 252 toward the inner side, the carrier 250 is disengaged from the plunger 240, and the carrier 250 may move relative to the plunger 240 in the third direction 3 by the elastic force of the needle spring 270b, which will be described later.

### Supporter

The supporter 260 may be coupled to the base cover 212. The supporter 260 may maintain the coupling between the transmitter 120 and the base cover 212 by engaging with the transmitter 120. The supporter 260 may release the coupling between the transmitter 120 and the base cover 212 by disengaging with the transmitter 120.

Referring to FIGS. 11, 13, 14, 18, and 19, the supporter 260 includes a pivot portion 261, a hook portion 262, an extension portion 263, and an expansion portion 264.

The pivot portion 261 is formed in a form extending in the first direction 1 and may be coupled to the support portion 2124 of the base cover 212. The pivot portion 261 may slide in the first direction 1 and may rotate about the first direction 1 as an axis while being coupled to the support portion 2124. As described above, the support portion 2124 may limit the movement of the pivot portion 261 along the second direction 2 (including the opposite direction to the second direction 2) and the movement along the third direction 3.

The hook portion 262 may be formed on one side of the pivot portion 261. By the rotation of the pivot portion 261 about the first direction 1 as an axis, the end portion of the hook portion 262 may be inserted into the housing groove 1216 of the transmitter 120, or may be pulled out from the housing groove 1216 of the transmitter 120.

The extension portion 263 may be formed on the other side of the pivot portion 262. Depending on whether the upper surface of the extension portion 263 and a stopper protrusion contact portion 2321 of the moving plate 230 to be described later are in contact, the rotation of the pivot portion 261 about the first direction 1 as an axis may be restricted or allowed. If the upper surface of the extension portion 263 is in contact with the stopper protrusion contact portion 2321, the rotation of the pivot portion 261 about the first direction 1 as an axis may be restricted. In this case, the movement of the supporter 260 may be restricted by a stopper protrusion 232, so that the coupling between the transmitter 120 and the base cover 212 may be maintained. If the upper surface of the extension portion 263 is not in contact with the stopper protrusion contact portion 2321, the rotation of the pivot portion 261 about the first direction 1 as an axis may be allowed. In this case, when the user moves the applicator 200 in the third direction 3 with respect to the sensing device 100 attached to the skin, the pivot portion 261 rotates about the first direction 1 as an axis so that the end portion of the hook portion 262 may come out from the housing groove 1216 of the transmitter 120.

The expansion portion 264 may be formed at each of the two end portions of the pivot portion 261 facing each other in the first direction 1. The diameter of the expansion portion 264 may be larger than the diameter of the pivot portion 261. Even if an external force is applied to the supporter 260 in the opposite direction to the first direction 1 after the supporter 260 is moved in the first direction 1, the expansion portion 264 may restrict the movement of the supporter 260 in the opposite direction to the first direction 1.

### Moving plate

The moving plate 230 may be disposed to be movable in the first direction 1 (including the opposite direction to the first direction 1) on the middle frame 221. The moving plate 230 may prevent movement of the sensor unit 110, the plunger 240, and the carrier 250 in the opposite direction to the third direction 3 in the deactivation position. The moving plate 230 may allow movement of the sensor unit 110, the plunger 240, and the carrier 250 in the opposite direction to the third direction 3 in the activation position. Movement of the moving plate 230 from the deactivation position to the activation position (i.e., movement of the moving plate 230 along the first direction 1) may be prevented or allowed by the locking unit 2214 of the middle frame 211. The moving plate 230 may limit and allow rotation of the supporter 260 about the first direction 1 as an axis.

Referring to FIGS. 11, 15, and 18, the moving plate 230 includes a moving plate body 2301, a moving plate opening 2302, a moving plate detent 2303, a prop portion 2304, and a stopper 2305.

The moving plate body 2301 may be disposed on the upper surface of the middle frame body 2210. The moving plate body 2301 may have an upper surface and a lower surface facing each other in the third direction 3, and may be formed in a plate shape overall.

The moving plate opening 2302 may be formed by penetrating the central portion of the moving plate body 2301. The moving plate opening 2302 may be in communication with the middle frame opening 2211. The column member 222 may be inserted into the moving plate opening 2302. The column member 222 may be coupled to the base cover 212 through the moving plate opening 2302 and the middle frame opening 2211.

The moving plate detent 2303 may be in communication with the moving plate opening 2302 by penetrating one side of the moving plate body 2301. A hook portion 2217 of the locking unit 2214 may be disposed in the moving plate detent 2303. As described above, depending on whether the leg portion 2218 of the locking unit 2214 and the removing unit 300 are in contact, the moving plate detent 2303 and the hook portion 2217 may be engaged or disengaged with each other. In a state where the moving plate detent 2303 and the hook portion 2217 are engaged with each other, the moving plate 230 cannot move in the first direction 1. In other words, in this case, even if the user presses the button portion 291 in the first direction 1, the moving plate 230 does not move from the deactivation position to the activation position. The moving plate 2300 may move from the deactivation position to the activation position only when the engagement between the moving plate detent 2303 and the hook portion 2217 is disengaged.

The prop portion 2304 may be formed to protrude on the upper surface of the moving plate body 2301. Specifically, the prop portion 2304 may be disposed on the periphery of the moving plate opening 2302. When the moving plate 230 is positioned in the deactivation position, the prop portion 2304 may support a plunger protrusion 242 to be described later to prevent movement of the plunger 240 along the opposite direction to the third direction 3. When the moving plate 230 is moved to the activation position, the prop portion 2304 may not support the plunger protrusion 242 to allow movement of the plunger 240 along the opposite direction to the third direction 3.

The stopper 2305 may be formed to protrude from the lower surface of the moving plate body 2301. The stopper 2305 may be formed on both sides of the moving plate body 2301 facing in the second direction 2. The stopper 2305 may be in contact with the supporter 260 to move the supporter 260 in the first direction 1 and prevent the supporter 260 from rotating about the first direction 1 as an axis. The stopper 2305 may be spaced apart from the supporter 260 to allow the supporter 260 to rotate about the first direction 1 as an axis. The stopper 2305 includes a stopper contact portion 2306 and a stopper detent 2307. The stopper contact portion 2306 may be a lower end portion region of the stopper 2305. The stopper detent 2307 may be extended in the opposite direction to the third direction 3 from one side of the stopper contact portion 2306. Hereinafter, the coupling and operation of the stopper 2305 and the supporter 260 will be described in more detail.

As shown in FIG. 18A and FIG. 19A, when the moving plate 230 is positioned in the deactivation position, the stopper contact portion 2306 contacts the extension portion 263 of the supporter 260, thereby restricting the rotation of the supporter 260 about the first direction 1 as an axis. At this time, the hook portion 262 of the supporter 260 may be engaged with the housing groove 1216 of the transmitter 120, thereby maintaining the coupling relationship between the transmitter 120 and the applicator 200. At this time, the frame retention protrusion 2128 may contact the side portion of the pivot portion 261 and the expansion portion 264 of the supporter 260.

As shown in FIG. 18B and FIG. 19B, when the moving plate 230 moves in the first direction 1 by the user's manipulation of the release button 290, the stopper detent 2307 of the moving plate 230 moves the supporter 260 in the first direction 1. At this time, the expansion portion 264 of the supporter 260 may pass through the frame retention protrusion 2128 while elastically deforming the frame retention protrusion 2128.

Referring to FIG. 19C, when the user's pressure on the button portion 291 of the release button 290 in the first direction 1 is removed, the engagement between the moving plate 230 and the supporter 260 is released. Specifically, when the user's pressure in the first direction 1 on the button portion 291 is removed, the moving plate 230 moves in the opposite direction to the first direction 1 by the elastic force of the elastic pressing portion 2219 (see FIGS. 12 and 15), but the supporter 260 is prevented from moving in the opposite direction to the first direction 1 by the frame retention protrusion 22128. As a result, the stopper 2305 of the moving plate 230 is detached from the extension portion 263 of the supporter 260. As a result, the supporter 260 may rotate about the first direction 1 as an axis, and the transmitter 120 may be detached from the applicator 200.

### Plunger

The plunger 240 may be disposed within the inner frame 220. The plunger 240 may be disposed so as to be movable in the opposite direction to the third direction 3 with respect to the inner frame 220. The plunger 240 may be engaged with the sensor unit 110 and the carrier 250 to be described later, and may move together in the opposite direction to the third direction 3.

Referring to FIGS. 11, 12, 15, 17, 18, and 21, the plunger 240 includes a plunger body 241, a plunger protrusion 242, a plunger detent 243, a plunger bottom portion 244, a plunger penetration hole 245, a pressing protrusion 246a, a guide protrusion 246b, a plunger fixing portion 247, a plunger coupling groove 248, and a plunger latch 249.

The plunger body 241 forms the overall appearance of the plunger 240, and a space for accommodating the carrier 250 may be provided inside. One side portion among the two side portions (one side portion and the other side portion) of the plunger body 241 facing in the first direction 1 may be open. The carrier 250 to be described later may be accommodated in the accommodation space inside the plunger body 241 through the open side portion of the plunger body 241.

The plunger protrusion 242 may be formed to protrude on each outer surface of the plunger body 241 facing in the second direction 2. When the plunger protrusion 242 contacts the prop portion 2304 of the moving plate 230, movement of the plunger 240 in the opposite direction to the third direction 3 may be prevented (see FIG. 18A). When the moving plate 230 moves in the first direction 1 and the plunger protrusion 242 and the prop portion 2304 do not contact each other, movement of the plunger 240 in the opposite direction to the third direction may be allowed (see FIG. 18B).

The plunger detent 243 may be formed on each inner surface of the plunger body 241 facing in the second direction 2. The plunger detent 243 may be engaged with a carrier latch 2523 of the carrier 250 to be described later. When the plunger detent 243 and the carrier latch 2523 are engaged, the carrier 250 cannot move relative to the plunger 240. When the plunger detent 243 and the carrier latch 2523 are disengaged, the carrier 250 may move relative to the plunger 240.

The plunger bottom portion 244 may be a surface of the outer surface of the plunger body 241 that contacts the sensor unit 110. The plunger penetration hole 245 may be formed by penetrating the plunger bottom portion 244 of the plunger body 241. The needle 280 to be described later may penetrate the sensor unit 110 coupled to the plunger bottom portion 244 through the plunger penetration hole 245.

Each of the pressing protrusion 246a and the guide protrusion 246b may be formed to protrude from the plunger bottom portion 244. The pressing protrusion 246a may be formed to be spaced apart from each other in the second direction 2 on one side portion of the plunger bottom portion 244. The guide protrusion 246b may be formed in the central portion in the second direction 2 of the other side portion of the plunger bottom portion 244. When coupling the sensor unit 110 with the plunger bottom portion 244, the pressing protrusion 246a may be inserted into the sensor unit groove 1134 (see FIGS. 3 and 7) of the sensor unit 110, and the guide protrusion 246b may be inserted into the cap coupling groove 1136 (see FIGS. 3 and 7) of the sensor unit 110. By inserting the pressing protrusion 246a and the guide protrusion 246b into the sensor unit groove 1133 and the cap coupling groove 1136 of the sensor unit 110, respectively, the position of the sensor unit 110 may be guided during coupling, and relative movement of the sensor unit 110 may be prevented after coupling.

The plunger fixing portion 247 (see FIG. 21) may be disposed on the inner side of the plunger body 241 to fix the needle spring 270b to the plunger body 241. The plunger fixing portion 247 includes a fixing portion groove 2471, a contact surface 2472, a barrier portion 2473, and an inlet 2474. The plunger fixing portion 247 may support movement of the carrier 250 along the third direction 3 by the elastic force of the needle spring 270b to be described later.

The plunger coupling groove 248 may be formed on the upper surface side among the outer surface of the plunger body 241. When movement of the plunger 240 along the opposite direction to the third direction 3 is completed, the plunger coupling groove 248 may be engaged with the blocking portion 2224 of the column member 222. When the plunger coupling groove 248 and the blocking portion 2224 are engaged with each other, the plunger coupling groove 248 may prevent movement of the plunger 240 along the third direction 3.

The plunger latch 249 may be formed on the outer surface of the other side portion of the plunger body 241. The plunger latch 249 engages with the clamping portion 2222 of the column member 222 during the assembling process of the applicator 200, and when the assembling process of the applicator 200 is completed, the engagement with the clamping portion 2222 is released. As described above, by engaging the plunger latch 249 and the clamping portion 2222 during the assembling process of the applicator 200, movement of the plunger 240 that is not intended by the operator may be prevented during the assembling process.

### Carrier

The carrier 250 may be disposed inside the plunger 240. When the carrier 250 and the plunger 240 are engaged, the carrier 250 may move in the opposite direction to the third direction 3 together with the plunger 240. When the carrier 250 and the plunger 240 are disengaged, the carrier 250 may move in the third direction 3 with respect to the plunger 240 by the elastic force of the needle spring 270b to be described later.

Referring to FIG. 15, the carrier 250 includes a carrier body 251, a carrier wing 252, and a grip arm 253.

The carrier body 251 may be formed in an overall hexahedral shape. The needle 280 may be coupled inside the carrier body 251. A carrier fixing portion 2511 to which the needle spring 270b to be described later is coupled may be formed on the upper portion of the carrier body 251.

The carrier wing 252 may be formed to extend in the third direction 3 from each of the two end surfaces (one end surface and the other end surface) of the carrier body 251 facing each other in the second direction 2. The carrier wing 252 may be engaged with or disengaged from the plunger 240. The carrier wing 252 includes a wing body 2521 that is elastically deformably connected to the carrier body 251, a trigger 2522 that protrudes toward the outer side from the wing body 2521, and the carrier latch 2523. The carrier latch 2523 may be engaged with the plunger detent 243 to prevent relative movement of the carrier 250 with respect to the plunger 240. In the process in which the plunger 240 and the carrier 250 move together in the opposite direction to the third direction 3, the trigger 2522 may be pressed toward the inner side by contacting the release portion 2226 of the column member 222 (see FIG. 23). When the trigger 2522 is pressed toward the inner side, the wing body 2521 may be elastically deformed toward the inner side, so that the carrier latch 2523 may come out of the plunger detent 243.

The grip arm 253 may be formed on each of the two side portions of the carrier body 251 facing in the first direction 1 to be elastically deformable in the first direction 1 (including the opposite direction to the first direction 1). For example, the grip arm 253 may include a horizontal extension portion extending toward the outer side of the carrier body 251 from each of the two side surfaces (one side surface and the other side surface) facing in the first direction 1 of the carrier body 251, and a vertical extension portion extending in the opposite direction to the third direction 3 from the end portion of the horizontal extension portion. The vertical extension portion of the grip arm 253 may be spaced apart from the two side surfaces of the carrier body 251 by the horizontal extension portion, and be elastically deformed with respect to an applied external force. A plurality of grip arms 253 may be formed spaced apart from each other on each side portion of the carrier body 251. The grip arm 253 may be capable of coupling and detaching the sensor unit 110 to and from the carrier 250. The grip arm 253 is provided with a grip catching portion 2531 protruding toward the inner side of the carrier body 251 from the lower end portion of the grip arm 253, and a grip protrusion portion 2532 protruding toward the outer side of the carrier body 251 on the outer surface of the grip arm 253. When the carrier 250 is located at the first location (meaning the state shown in FIG. 21), the grip protrusion portion 2532 comes into contact with the inner wall of the column member 222, so that the grip arm 253 may be elastically deformed toward the inner side of the carrier body 251. Accordingly, the grip catching portion 2531 moves toward the sensor unit housing 112 and comes into contact with the cap fence 1138 (see FIG. 21). As a result, the carrier 250 and the sensor unit 110 may remain a coupled state. When the sensor unit 110 and the carrier 250 are moved in the opposite direction of the third direction 3 by the plunger 240, the grip protrusion portion 2532 is released from the inner wall of the column member 222, and the grip arm 253 may be elastically deformed toward the outer side of the carrier body 251. Accordingly, the grip catching portion 2531 is moved toward the outer side of the sensor unit housing 112, and the coupling force between the grip arm 253 and the sensor unit 110 is weakened. The sensor unit 110 is coupled to the transmitter 120 by the adhesive layer 123, and in this state, when the carrier 250 is pulled in the third direction 3 by the needle spring 270b, the grip arm 253 may be detached from the sensor unit 110.

### Needle

The needle 280 is coupled to the carrier 250 and may assist the transcutaneous sensor 111 in inserting the insertion portion 1116 of the transcutaneous sensor 111 into the user's skin. To this end, the needle 280 may be moved in the opposite direction to the third direction 3 together with the sensor unit 110 in a state of being coupled to the sensor unit 110 and inserted into the user's skin. In addition, the needle 280 may be moved in the third direction 3 together with the carrier 250 and detached from the sensor unit 110 after the movement in the opposite direction to the third direction 3 is completed or during the movement.

Referring to FIG. 15, the needle 280 includes a needle body 281, a needle head 282, and a needle coupler 283.

The needle body 281 may be formed into a shape with a sharp end so that it may be smoothly inserted into the user's skin by piercing the user's skin. The needle body 281 may guide the skin insertion of the transcutaneous sensor 111 by piercing the user's skin before the insertion portion 1116 of the transcutaneous sensor 111 when the sensor unit 110 moves in the opposite direction to the third direction 3. The needle body 281 may form an internal space with one side open to accommodate the insertion portion 1116 of the transcutaneous sensor 111 in the internal space. The needle head 282 may extend upward from the needle 280. The needle head 282 may be coupled to the needle coupler 283 to support the needle body 281. The needle coupler 283 may be fixed to the inner side of the carrier 250 to fix the needle head 282 to the carrier 250.

### Needle spring

The needle spring 270b may be disposed between the plunger 240 and the needle 280. Specifically, one end of the needle spring 270b may be coupled with the plunger fixing portion 247, and the other end of the needle spring 270b may be coupled with the carrier fixing portion 2511 to which the needle 280 is coupled (see FIG. 21). The needle spring 270b provides a driving force for movement of the carrier 250 in the third direction 3, so that the carrier 250 may move relative to the plunger 240 by the needle spring 270b. The needle spring 270b is an elastic member in a tensioned spring form and may apply an elastic force to the carrier 250 in a direction away from the sensor unit 110. In other words, the needle spring 270b may move the carrier 250 and the needle 280 so that the carrier 250 and the needle 280 may be detached from the sensor unit 110.

Referring to FIG. 21, one end of the needle spring 270b is coupled to the plunger fixing portion 247. In the process of assembling the needle spring 270b to the plunger 240, one end of the needle spring 270b may be disposed inside the fixing portion groove 2471 through the inlet 2474. One end of the needle spring 270b comes into close contact with the contact surface 2472 in a state being disposed inside the fixing portion groove 2471. The barrier portion 2473 may prevent one end of the needle spring 270b disposed inside the fixing portion groove 2471 from coming out of the fixing portion groove 2471. The other end of the needle spring 270b is fixed to the carrier fixing portion 2511 of the carrier 250. The carrier fixing portion 2511 of the carrier 250 may also have the same configuration as the plunger fixing portion 247 of the plunger 240, or may have a different configuration. The needle spring 270b coupled to the carrier fixing portion 2511 and the plunger fixing portion 247 is a coil spring, and may maintain a tensioned state when the plunger 240 and the carrier 250 are engaged. When the engagement between the plunger 240 and the carrier 250 is released, the needle spring 270b may contract due to elastic force to move the needle 280 in the third direction 3. Although the embodiment using the tensile force of the coil spring has been described, the scope of the present disclosure is not limited thereto.

### Plunger spring

The plunger spring 270a may be disposed between the column member 222 and the plunger 240. The plunger spring 270a may provide a driving force for movement of the plunger 240 in the opposite direction to the third direction 3 with respect to the column member 222. The plunger spring 270a may be an elastic member that provides elastic force. One end of the plunger spring 270a may be coupled to the column member 222, and the other end of the plunger spring 270a may be coupled to the plunger 240 on the inner side of the column member 222 in a compressed state. When the moving plate 230 is moved in the first direction 1 so that the plunger protrusion 242 and the prop portion 2304 do not come into contact, the plunger spring 270a may expand by the elastic force and move the plunger 240 in the opposite direction to the third direction 3. The plunger spring 270a may be a coil spring, but the scope of the present disclosure is not limited thereto.

### 4. Removing unit

The removing unit 300 may be coupled with the applicator 200 to restrict the operation of the release button 290. In other words, the removing unit 300 may prevent the moving plate 230 from moving in the first direction 1 even if the release button 290 is pressed in the first direction 1 due to an unintentional action of the user (preventing movement from the deactivation position to the activation position). The removing unit 300 may be moved together with the protective sheet 400 when it is removed from the applicator 200 through the entrance 2126 of the main frame 210, thereby detaching the protective sheet 400 from the adhesive layer 123.

Referring to FIGS. 14 and 20, the removing unit 300 includes a removing body 310 and a holder 320.

The removing body 310 may restrict the movement of the moving plate 230 in a state being disposed on the inner side of the main frame 210. Specifically, when the moving plate 230 is positioned in the deactivation position and the removing unit 300 is positioned on the inner side of the main frame 210, the removing body 310 comes into contact with the leg portion 2218 of the locking unit 2214, and elastic deformation of the body portion 2216 is not allowed. At this time, the hook portion 2217 of the locking unit 2214 engages with the moving plate detent 2303 of the moving plate 230. In this state, movement of the moving plate 230 in the first direction 1 is not allowed. Meanwhile, when the removing unit 300 is pulled out to the entrance 2126 and removed from the main frame 210, elastic deformation of the body portion 2216 is allowed, so that the engagement between the hook portion 2217 and the moving plate detent 2303 may be released. In this state, when the release button 290 is pressed in the first direction 1, the body portion 2216 of the locking unit 2214 is elastically deformed, so that the engagement between the hook portion 2217 and the moving plate detent 2303 is released, and the moving plate 230 is moved in the first direction 1.

An insertion hole 311 and a sliding hole 312 are formed in the removing body 310. The wing portion 420 of the protective sheet 400 may be inserted into the insertion hole 311. The sliding hole 312 may be formed in a shape extending in the movement direction of the holder 320, so as to guide the movement of the holder 320 relative to the removing body 310.

A handle portion 313 is disposed on one end portion of the removing body 310, and a moving tab engaging portion 314 is disposed on the other end of the removing body 310. When the removing unit 300 is located on the inner side of the main frame 210, the handle portion 313 is disposed on the outer side of the main frame 210. The user may detach the removing unit 300 from the main frame 210 using the handle portion 313. The moving tab engaging portion 314 may engage with the engaging protrusion 2127 when the removing unit 300 is located on the inner side of the main frame 210 (see FIG. 14). When a certain amount of force is applied to the removing unit 300 in the opposite direction of the first direction 1, the moving tab engaging portion 314 may be disengaged from the engaging protrusion 2127.

The holder 320 is inserted into the sliding hole 312 and movably coupled to the removing body 310. The holder 320 includes a holding portion 321 and a pressing portion 322. The holding portion 321 may couple the protective sheet 400 to the removing unit 300. Specifically, when the holding portion 321 is inserted into the protective sheet hole 421 in a state where the wing portion 420 of the protective sheet 400 is inserted into the insertion hole 311 of the removing body 310, the protective sheet 400 may be coupled to the removing unit 300. The pressing portion 322 may protrude from the removing body 310 and press the protective sheet 400 in the opposite direction to the third direction 3. Specifically, when the removing unit 300 is located on the inner side of the main frame 210, the pressing portion 322 may press the protective portion 410 of the protective sheet 400 toward the adhesive layer 123. The pressing portion 322 may be formed of an elastically deformable material and structure so that the removing unit 300 may pass through the entrance 2126 when removed from the applicator 200.

### 5. Release paper

The release paper 500 may be attached to the lower surface of the adhesive portion 124 to prevent external exposure of the adhesive portion 124. The release paper 500 may be detached from the adhesive portion 124 by the user, thereby exposing the lower surface of the adhesive portion 124 to the outside. For example, the release paper 500 may be a single member or multiple members. In the latter case, a plurality of release papers 500 may include a first release paper covering one region of the lower surface of the adhesive paper 124 and a second release paper covering the remainder of the lower surface of the adhesive paper 124. In this case, the first release paper and the second release paper may overlap each other, but the scope of the present disclosure is not limited thereto.

### 6. Sensing device attachment process

Hereinafter, a process of attaching the sensing device 100 to the user's skin using the applicator assembly 10 according to an embodiment of the present disclosure will be described.

First, as shown in FIG. 21, the removing unit 300 is removed from the applicator 200. When the removing unit 300 is moved by the user in the opposite direction to the first direction 1, the protective sheet 400 coupled with the removing unit 300 is detached from the adhesive layer 123 of the transmitter 120. In a state before the removing unit 300 is removed from the applicator 200, the removing unit 300 is in contact with the leg portion 2218 of the middle frame 221, and thus the hook portion 2217 is biased in the direction of engaging with the moving plate 230. When the removing unit 300 is removed from the applicator 200 by the user, the leg portion 2218 may be biased in the opposite direction of the third direction 3, and thus the hook portion 2217 is biased to disengage from the release button 290.

Next, the release paper 500 (see FIG. 1) is removed from the adhesive portion 124 of the transmitter 120 to expose the adhesive portion 124, and the applicator 200 is disposed so as to contact the user's skin via the adhesive portion 124. Meanwhile, unlike the description above, an operation prevention portion 40 may be removed after the release paper 500 is removed.

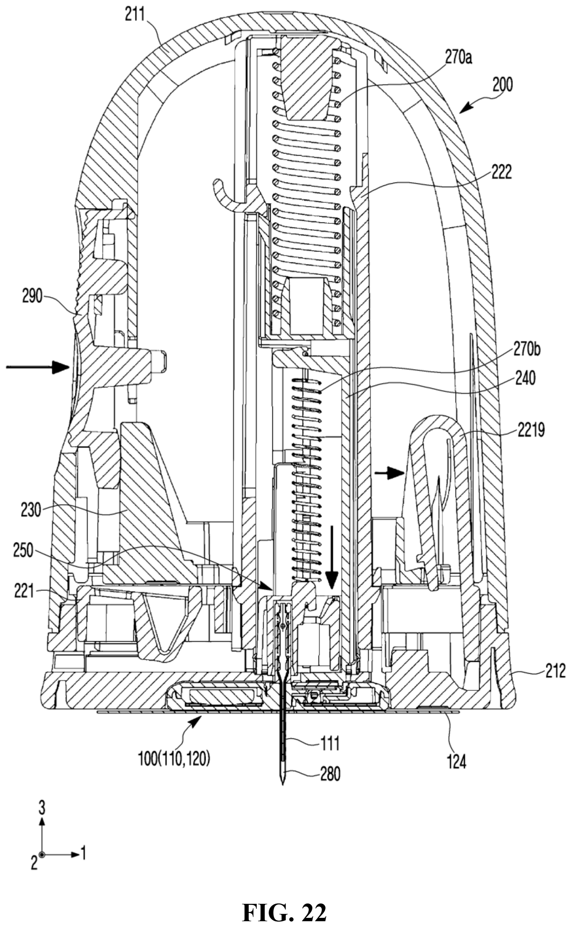

Next, as illustrated in FIG. 22, the release button 290 is pressed to move the moving plate 230 in the first direction 1. Accordingly, movement of the sensor unit 110, the plunger 240, and the carrier 250 in the opposite direction to the third direction 3 is allowed. Specifically, when the release button 290 is pressed by the user in the first direction 1, the moving plate 230 moves in the first direction 1 while elastically deforming the elastic pressing portion 2219 to be positioned at the activation position. As the moving plate 230 moves to the activation position, the plunger protrusion 242 of the plunger 240 is disengaged from the prop portion 2304 of the moving plate 230 (see FIGS. 18A and 18B). Accordingly, as illustrated in FIG. 22, the plunger 240 is moved in the opposite direction to the third direction 3 by the expansion of the plunger spring 270a. As the plunger 240 is moved in the opposite direction to the third direction 3, the needle 280 and the transcutaneous sensor 111 are inserted into the user's skin, and the sensor unit 110 is attached to the transmitter 120 by the adhesive layer 123.

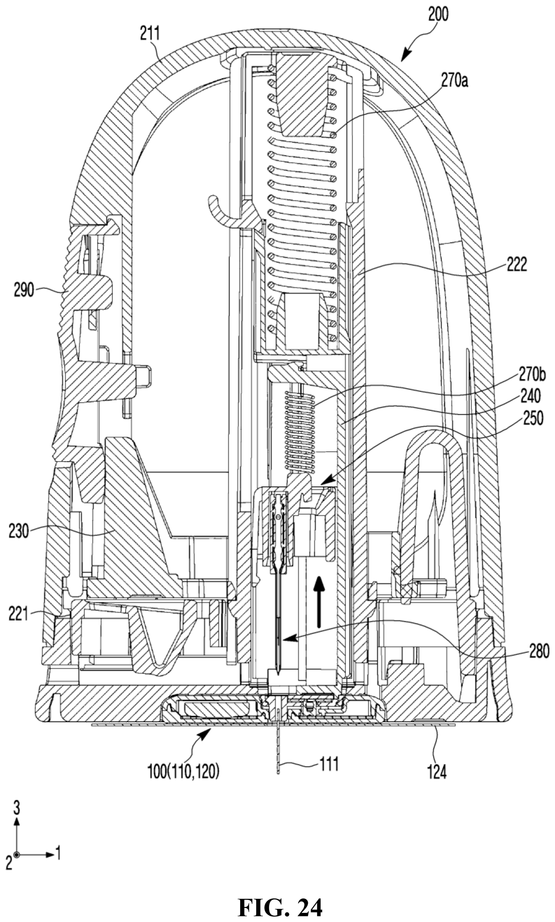

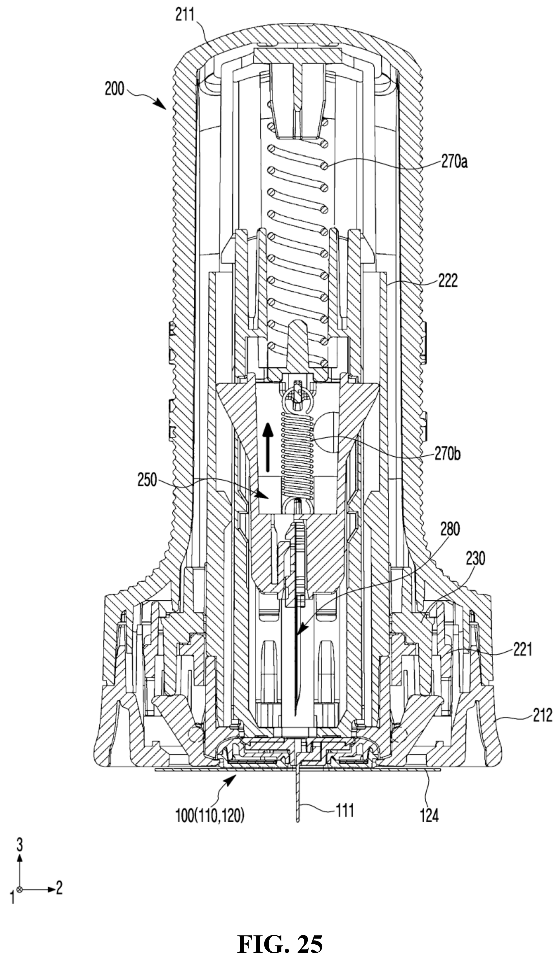

Meanwhile, as illustrated in FIG. 23, when the plunger 240 is moved in the opposite direction to the third direction 3, the release portion 2226 of the column member 222 comes into contact with the trigger 2522 of the carrier 250 to elastically deform the wing body 2521. As a result, the carrier latch 2523 (see FIG. 15) is disengaged from the plunger detent 243 (see FIG. 15). When the carrier 250 is disengaged from the plunger 240, as illustrated in FIGS. 24 and 25, the carrier 250 is moved in the third direction 3 away from the user's skin by the contraction of the needle spring 270b. As a result, the needle 280 is pulled out of the user's skin. Meanwhile, while the carrier 250 and the needle 280 are moved in the third direction 3, the sensing device 100 may remain the attached state to the user's skin by the adhesive portion 124, and the plunger bottom portion 244 of the plunger 240 may remain the contact state with the sensor unit 110.

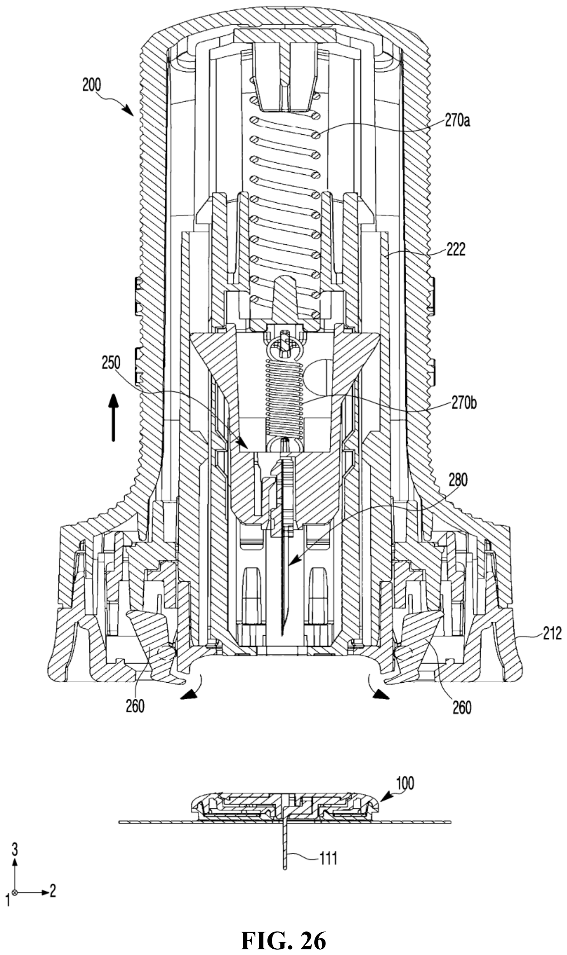

After the transcutaneous sensor 111 is inserted into the user's skin, the applicator 200 may be detached from the sensing device 100 attached to the skin. As described above, when movement of the moving plate 230 to the activation position is completed, the supporter 260 engaged with the transmitter 120 becomes rotatable about the first direction 1 as an axis. Accordingly, as illustrated in FIG. 26, when the user lifts the applicator 200 in the third direction 3, the supporter 260 is rotated, and the applicator 200 is detached from the sensing device 100.

Although embodiments of the present disclosure have been described above, those skilled in the art will be able to make various modifications and changes to the present disclosure by adding, changing or deleting components, without departing from the spirit of the present disclosure as set forth in the appended claims, which are included within the scope of rights of the present disclosure.
10: Applicator assembly
100: Sensing device
110: Sensor unit
120: Transmitter
200: Applicator
210: Main frame
220: Inner frame
230: Moving plate
240: Plunger
250: Carrier
260: Supporter
270a, 207b: Spring
280: Needle
290: Release button
300: Removing Unit
400: Protective sheet
500: Release paper

## Claims

1. A sensing device (100) to be inserted and attached to a user through an applicator (200), the sensing device (100) comprising:
a sensor unit (110) comprising a transcutaneous sensor (111), a sensor unit housing (112) configured to accommodate the transcutaneous sensor (111) inside while exposing one end of the transcutaneous sensor (111) through a lower surface, and a base leg (1128) protruding from the lower surface of the sensor unit housing (112); and
a transmitter (120) disposed at a lower part of the sensor unit (110) within the applicator (200),
wherein the transmitter (120) comprises:
a transmitter housing (121) having a mounting portion (1212) formed on an upper surface and configured to accommodate the sensor unit (110); and
a coupling protrusion (1215) disposed on the mounting portion (1212) and configured to be engaged with the base leg (1128).

2. The sensing device of claim 1, wherein an exposure hole (1214) configured to expose an inside of the transmitter housing (121) is formed on a bottom surface of the mounting portion (1212), and
the coupling protrusion (1215) comprises a support portion (1215-1) in contact with the bottom surface of the mounting portion (1212), and a protrusion portion (1215-2) extending from the support portion (1215-1) to protrude from an inner wall of the exposure hole (1214).

3. The sensing device of claim 2, wherein a distance by which a lower region of the protrusion portion (1215-2) protrudes from the inner wall of the exposure hole (1214) is longer than a distance by which an upper region of the protrusion portion (1215-2) protrudes from the inner wall of the exposure hole (1214).

4. The sensing device of claim 2, wherein the base leg (1128) comprises:
leg portions (1128-1) respectively in contact with the lower surface of the sensor unit housing (112) and spaced apart from each other; and
a connecting portion (1128-2) configured to connect lower end portions of the leg portions (1128-1) spaced apart from each other and define a fastening hole (1128H) together with the leg portions (1128-1).

5. The sensing device of claim 4, wherein a thickness of an upper end portion of the connecting portion (1128-2) connected to the leg portions is thicker than a thickness of a lower end portion of the connecting portion (1128-2).

6. The sensing device of claim 5, wherein a thickness of the connecting portion (1128-2) increases from the lower end portion of the connecting portion (1128-2) toward the upper end portion of the connecting portion (1128-2).

7. A sensing device (100) comprising:
a sensor unit (110) comprising a transcutaneous sensor (111), a sensor unit housing (112) configured to accommodate the transcutaneous sensor (111) inside while exposing one end of the transcutaneous sensor (111) through a lower surface, and a base leg (1128) protruding from the lower surface of the sensor unit housing (112); and
a transmitter (120) coupled with the sensor unit (110),
wherein the transmitter (120) comprises:
a transmitter housing (121);
a mounting portion (1212) formed on the transmitter housing (121) and configured to accommodate the sensor unit housing (112);
an insertion hole (1213) penetrating the transmitter housing (121) and configured to accommodate at least a portion of one end of the transcutaneous sensor (111); and
a coupling protrusion (1215) disposed within the mounting portion (1212) and configured to be engaged with the base leg (1128).

8. The sensing device of claim 7, wherein the base leg (1128) comprises:
leg portions (1128-1) respectively in contact with the lower surface of the sensor unit housing (112) and spaced apart from each other; and
a connecting portion (1128-2) configured to connect lower end portions of the leg portions (1128-1) spaced apart from each other and define a fastening hole (1128H) together with the leg portions (1128-1), and
wherein at least a portion of the coupling protrusion (1215) is inserted into the fastening hole (1128H).

9. The sensing device of claim 8, wherein an exposure hole (1214) configured to accommodate the base leg (1128) is formed on a bottom surface of the mounting portion (1212), and
a distance from one surface of the leg portion (1128-1) facing an inner wall of the exposure hole (1214) to the inner wall of the exposure hole (1214) is shorter than a distance from one surface of the connecting portion (1128-2) facing the inner wall of the exposure hole (1214) to the inner wall of the exposure hole (1214).

10. The sensing device of claim 8, wherein an exposure hole (1214) configured to accommodate the base leg (1128) is formed on a bottom surface of the mounting portion (1212), and
the coupling protrusion (1215) comprises a support portion (1215-1) in contact with the bottom surface of the mounting portion (1212), and a protrusion portion (1215-2) extending from the support portion (1215-1) to protrude from an inner wall of the exposure hole (1214).

11. The sensing device of claim 10, wherein a distance by which the protrusion portion (1215-2) protrudes from an inner wall of the exposure hole (1214) is longer at a lower side of the inner wall of the exposure hole (1214) than at an upper side of the inner wall of the exposure hole (1214).

12. The sensing device of claim 7, further comprising an adhesive layer (123) adhered to each of a bottom surface of the mounting portion (1212) and a lower surface of the sensor unit housing(112).
